# EUROPEAN PATENT APPLICATION

(11) **EP 4 458 843 A1**
(43) Date of publication of application: **06.11.2024**
(21) Application number: 22915221.0
(22) Date of filing: 30.12.2022
(51) Int. Cl.: C07K 14/08, A61K 39/12, C07K 16/10, A61K 39/39

(54) **MRNA VACCINE**

(30) Priority: 31.12.2021 CN 202111683458
(71) Applicant: Guangzhou National Laboratory, Guangzhou, Guangdong 510005 (CN)
(72) Inventor: PENG, Hua, Guangzhou, Guangdong 510005 (CN); CAO, Xuezhi, Guangzhou, Guangdong 510005 (CN); WANG, Xiuye, Guangzhou, Guangdong 510005 (CN)
(74) Representative: Biggi, Cristina
(86) International application number: PCT/CN2022/144148
(87) International publication number: WO 2023/125974

(57) **Abstract**

Provided is an mRNA vaccine, said mRNA vaccine containing an immune cell targeting molecule that is expressed in fusion with an antigen and enhances the immunological effectiveness of an mRNA vaccine.

## Description

### TECHNICAL FIELD

The present application relates to the field of vaccine development and, more specifically, relates to mRNA vaccines and their use thereof.

### BACKGROUND

Messenger ribonucleic acid (mRNA) is a single-stranded RNA molecule corresponding to a genetic sequence of a gene, which is translated by ribosomes during the process of protein synthesis in organisms to produce proteins. mRNA transcription can be carried out by combining T7 bacteriophage RNA polymerase with plasmid DNA. An mRNA transcribed *in vitro* has the same structural components as natural mRNA in a eukaryotic cell: a 5' cap, a 5' untranslated region (5' UTR), an open reading frame (ORF) encoding the relevant antigen, a 3' untranslated region (3' UTR), and a 3' poly A tail [3' Poly(A)]. Using synthetic 5'-cap analogs can improve mRNA stability and increase protein translation. An mRNA nucleotide can be modified to reduce innate immune activation and increase mRNA half-life in a host cell.

An mRNA vaccine is a vaccine that utilizes mRNA molecules encapsulated in lipid nanoparticles (LNP) to generate immune responses. The mRNA can be delivered to an immune cell but also to other cells, such as a muscle cell or an epithelial cell. The proteins translated by these cells can be expressed inside the cell, on the surface of the cell membrane, or secreted outside the cell, and finally targeted to an immune cell. Once a host cell produces an exogenous antigen, adaptive immune responses will be induced. The antigen is broken down by the proteasome, and MHC class I and class II molecules attach to the antigen and transport it to a cell membrane to activate a dendritic cell. Once activated, a dendritic cell migrates to lymph nodes, where they present antigens to T cells and B cells, ultimately leading to immune responses. The advantages of an mRNA vaccine are that it is easy to design, can present multiple antigens, has the potential to develop multi-linked and multivalent vaccines, has a short production cycle, and is easier to expand production capacity. Besides, it will not integrate with human DNA and has no risk of exogenous virus infection.

Therefore, mRNA vaccines are the third-generation vaccines after inactivated, live attenuated, subunit, and viral vector vaccines. The mRNA vaccine has the characteristics of fast switching to mutant pathogens, simple production process, and easy scale-up. However, the toxicity and side effects of an mRNA vaccine are also pronounced, such as sore reaction at the injection site, fatigue, headache, and even facial paralysis. The side effects are even more significant after a second injection. There are still many areas for optimization and improvement in the existing mRNA vaccine technology, and many technical barriers that need to be broken through. At present, the research on the improvement of mRNA vaccines mainly focuses on LNP delivery technology, production of plasmids, synthesis and purification of mRNA. Meanwhile, optimization of mRNA structure and the sequence itself mostly focuses on the modification of nucleotides encoding antigens, and there is no report that the open reading frame encoding the associated antigen additionally contains a coding sequence encoding an immune cell targeting molecule.

The present application provides an mRNA vaccine platform for enhancing immune responsiveness. The mRNA vaccine comprises an open reading frame, the open reading frame encodes an antigen and an immune cell targeting molecule. The mRNA vaccine with enhanced immune effect of the present application has the impact of enhancing the immune effect, improving the protection rate of the mRNA vaccine, reducing the dosage of the mRNA vaccine, enlarging the production capacity of the mRNA vaccine, and reducing the toxic and side effects of the mRNA vaccine.

In the first aspect, the present application provides an mRNA containing an open reading frame (ORF), wherein the open reading frame encodes an antigen (Antigen) and an immune cell targeting molecule. The antigen and the immune cell targeting molecule encoded by the open reading frame are a fusion protein of the antigen and the immune cell targeting molecule.

In some embodiments, the immune cell targeting molecule is one or more selected from the following:
A: an antibody or a polypeptide capable of binding to an immune cell surface protein;
B: a cytokine capable of activating an immune cell;
C: Pan epitope (PADRE) capable of activating an immune cell; and
D: an immunoglobulin Fc capable of binding to an immune cell.

In some embodiments, the open reading frame encodes a fusion protein of an antigen with A; a fusion protein of an antigen with A and B; a fusion protein of an antigen with A, B, and C; a fusion protein of an antigen with A, B, C and D; a fusion protein of an antigen with B; a fusion protein of an antigen with B and C; a fusion protein of an antigen with B, C and D; a fusion protein of an antigen with C; a fusion protein of an antigen with C and D; or a fusion protein of an antigen with D.

The fusion protein can be any arrangement of an antigen with A and/or B and/or C and/or D, for example, an antigen and A and/or B and/or C and/or D in the fusion protein are located at the C-terminus or N-terminus relatively to each other.

In some embodiments, the fusion protein is a homodimer or a heterodimer.

The mRNA provided according to the present application is capable of expressing a secreted fusion protein or a membrane fusion protein. In some embodiments, the secreted fusion protein expressed by the mRNA can be efficiently targeted to the surface of an immune cell because it contains an immune cell-targeting molecule.

The antibody or polypeptide A capable of binding to an immune cell surface protein includes, but not limited to, an antibody against CD274 (PDL1), PDCD1LG2 (PDL2), CLEC9A, LY75 (DEC205), CD40, TNFSF9 (4-1BB-L) and/or TNFSF4 (OX4OL), etc., or an active fragment of a ligand thereof.

The cytokine B capable of activating an immune cell includes, but not limited to, interleukin (IL), Colony-stimulating factor (CSF), and interferon (IFN).

Preferably, the interleukin comprises IL2, IL12, IL15, and IL21, or an active fragment thereof. IL2, or active fragment or homologue thereof is from human, cow, sheep, cat, canine, horse, rabbit, monkey, mouse, rat, alpaca, or camel.

Preferably, the colony-stimulating factor comprises CSF1, CSF2 and CSF3, or an active fragment thereof.

Preferably, the interferon comprises type I interferon, type II interferon, and type III interferon, or an active fragment thereof, wherein the type I interferon comprises IFNα, IFNω, IFNε, IFNκ, and IFNβ, or an active fragment thereof, the type II interferon comprises IFN-γ, or an active fragment thereof, and the type III interferon comprises IFN-λ1, IFN-λ2, IFN-λ3, and IFN-λ4, or an active fragment thereof.

Preferably, the Pan epitope (PADRE) C capable of activating an immune cell has an amino acid sequence as shown by AKFVAAWTLKAAA (SEQ ID NO: 1).

The immunoglobulin Fc (D) may be Fc from IgG, IgM, IgA, IgE or IgD, or a mutant thereof. The Fc is a mutant to form a heterodimeric protein.

In some embodiments, the mRNA further comprises a 5' UTR (an untranslated region) sequence and a 3' UTR (an untranslated region). The 5' UTR and 3' UTR can be the 5' UTR and 3' UTR of any highly expressed gene.

In some embodiments, the mRNA further comprises a 5' Cap.

In some embodiments, the mRNA further comprises a 3' Poly(A).

The antigen (Antigen) may be an immunogenic protein or an immunogenic fragment thereof capable of inducing an immune response against a pathogenic microorganism.

The pathogenic microorganism includes, but not limited to, SARS-Cov-2, SARS, cytomegalovirus (CMV), Herpes Simplex Virus-1 and -2, RSV, influenza virus, Ebola virus, Epstein-Barr virus (EBV), dengue virus, Zike virus, HIV, rabies virus, plasmodium gametocyte, herpes zoster virus (HZV), hepatitis B virus (HBV), hepatitis C virus (HCV), hepatitis D virus (HDV), HPV, *Mycobacterium tuberculosis,* or *Helicobacter pylori,* etc.

The antigen may be an immunogenic protein or an immunogenic fragment thereof capable of inducing an immune response against a cancer cell.

In some embodiments, the antigen may be a tumor antigen, such as MelanA/MART1, cancer-germline antigen, gp100, tyrosinase, CEA, PSA, Her-2/neu, survivin, telomerase, or an immunogenic fragment thereof.

In some embodiments, the open reading frame (ORF) further comprises a region encoding a linking fragment.

The antigen and the immune cell targeting molecule, and/or the immune cell targeting molecules are connected through a linking fragment.

In some embodiments, the linking fragment can be a flexible linking fragment, a rigid linking fragment, or an *in vivo* cleavage linking fragment. An amino acid sequence of the flexible linking fragment may be, but not limited to, (G)_{N}, (GS)_{N}, (GGS)_{N}, (GGGS)_{N}, or (GGGGS)_{N}.

In some embodiments, the mRNA has a structure selected from the group consisting of:
5' Cap-5' UTR-A-Antigen-3' UTR-3' Poly(A);
5' Cap-5' UTR-αPDL1-Antigen-3' UTR-3' Poly(A);
5' Cap-5' UTR-CLEC9A binding peptide-Antigen-3' UTR-3' Poly(A);
5' Cap-5' UTR-aDEC205-Antigen-3' UTR-3' Poly(A);
5' Cap-5' UTR-A-Linker-Antigen-3' UTR-3' Poly(A);
5' Cap-5' UTR-αPDL1-Linker-Antigen-3' UTR-3' Poly(A);
5' Cap-5' UTR-αPDL1-(GGGGS)₃-Antigen-3' UTR-3' Poly(A);
5' Cap-5' UTR-CLEC9A binding peptide-Linker-Antigen-3' UTR-3' Poly(A);
5' Cap-5' UTR-CLEC9A binding peptide-(GGGGS)₃-Antigen-3' UTR-3' Poly(A);
5' Cap-5' UTR-aDEC205-Linker-Antigen-3' UTR-3' Poly(A);
5' Cap-5' UTR-αDEC205-(GGGGS)₃-Antigen-3' UTR-3' Poly(A);
5' Cap-5' UTR-Antigen-A-3' UTR-3' Poly(A);
5' Cap-5' UTR-Antigen-αPDL1-3' UTR-3' Poly(A);
5' Cap-5' UTR-Antigen-CLEC9A binding peptide-3' UTR-3' Poly(A);
5' Cap-5' UTR-Antigen-CD40L-3' UTR-3' Poly(A);
5' Cap-5' UTR-Antigen-4-1BB-3' UTR-3' Poly(A);
5' Cap-5' UTR-Antigen-OX40-3' UTR-3' Poly(A);
5' Cap-5' UTR-Antigen-Linker-A-3' UTR-3' Poly(A);
5' Cap-5' UTR-Antigen-Linker-αPDL1-3' UTR-3' Poly(A);
5' Cap-5' UTR-Antigen-(GGGGS)₃-αPDL1-3' UTR-3' Poly(A);
5' Cap-5' UTR-Antigen-Linker-CLEC9A binding peptide-3' UTR-3' Poly(A);
5' Cap-5' UTR-Antigen-(GGGGS)₃-CLEC9A binding peptide-3' UTR-3' Poly(A);
5' Cap-5' UTR-Antigen-Linker-CD40L-3' UTR-3' Poly(A);
5' Cap-5' UTR-Antigen-(GGGGS)₃-CD40L-3' UTR-3' Poly(A);
5' Cap-5' UTR-Antigen-Linker-4-1BB-3' UTR-3' Poly(A);
5' Cap-5' UTR-Antigen-(GGGGS)₃-4-1BB-3' UTR-3' Poly(A);
5' Cap-5' UTR-Antigen-Linker-OX40-3' UTR-3' Poly(A);
5' Cap-5' UTR-Antigen-(GGGGS)₃-OX40-3' UTR-3' Poly(A);
5' Cap-5' UTR-B-Antigen-3' UTR-3' Poly(A);
5' Cap-5' UTR-IL2-Antigen-3' UTR-3' Poly(A);
5' Cap-5' UTR-IL12-Antigen-3' UTR-3' Poly(A);
5' Cap-5' UTR-IL15-Antigen-3' UTR-3' Poly(A);
5' Cap-5' UTR-IL21-Antigen-3' UTR-3' Poly(A);
5' Cap-5' UTR-CSF2-Antigen-3' UTR-3' Poly(A);
5' Cap-5' UTR-IFNa-Antigen-3' UTR-3' Poly(A);
5' Cap-5' UTR-B-Linker-Antigen-3' UTR-3' Poly(A);
5' Cap-5' UTR-IL2-Linker-Antigen-3' UTR-3' Poly(A);
5' Cap-5' UTR-IL2-(GGGGS)₃-Antigen-3' UTR-3' Poly(A);
5' Cap-5' UTR-IL12-Linker-Antigen-3' UTR-3' Poly(A);
5' Cap-5' UTR-IL12-(GGGGS)₃-Antigen-3' UTR-3' Poly(A);
5' Cap-5' UTR-IL15-Linker-Antigen-3' UTR-3' Poly(A);
5' Cap-5' UTR-IL15-(GGGGS)₃-Antigen-3' UTR-3' Poly(A);
5' Cap-5' UTR-IL21-Linker-Antigen-3' UTR-3' Poly(A);
5' Cap-5' UTR-IL21-(GGGGS)₃-Antigen-3' UTR-3' Poly(A);
5' Cap-5' UTR-CSF2-Linker-Antigen-3' UTR-3' Poly(A);
5' Cap-5' UTR-CSF2-(GGGGS)₃-Antigen-3' UTR-3' Poly(A);
5' Cap-5' UTR-IFNα-Linker-Antigen-3' UTR-3' Poly(A);
5' Cap-5' UTR-IFNα-(GGGGS)₃-Antigen-3' UTR-3' Poly(A);
5' Cap-5' UTR-Antigen-B-3' UTR-3' Poly(A);
5' Cap-5' UTR-Antigen-CSF2-3' UTR-3' Poly(A);
5' Cap-5' UTR-Antigen-Linker-B-3' UTR-3' Poly(A);
5' Cap-5' UTR-Antigen-Linker-CSF2-3' UTR-3' Poly(A);
5' Cap-5' UTR-Antigen-(GGGGS)₃-CSF2-3' UTR-3' Poly(A);
5' Cap-5' UTR-C-Antigen-3' UTR-3' Poly(A);
5' Cap-5' UTR-Pan-Antigen-3' UTR-3' Poly(A);
5' Cap-5' UTR-C-Linker-Antigen-3' UTR-3' Poly(A);
5' Cap-5' UTR-Pan-Linker-Antigen-3' UTR-3' Poly(A);
5' Cap-5' UTR-Pan-(GS)₃-Antigen-3' UTR-3' Poly(A);
5' Cap-5' UTR-Antigen-C-3' UTR-3' Poly(A);
5' Cap-5' UTR-Antigen-Pan-3' UTR-3' Poly(A);
5' Cap-5' UTR-Antigen-Linker-C-3' UTR-3' Poly(A);
5' Cap-5' UTR-Antigen-Linker-Pan-3' UTR-3' Poly(A);
5' Cap-5' UTR-Antigen-(GS)₃-Pan-3' UTR-3' Poly(A);
5' Cap-5' UTR-A-Antigen-B-3' UTR-3' Poly(A);
5' Cap-5' UTR-αPDL1-Antigen-CSF2-3' UTR-3' Poly(A);
5' Cap-5' UTR-A-Linker-Antigen-Linker-B-3' UTR-3' Poly(A);
5' Cap-5' UTR-αPDL1-Linker-Antigen-Linker-CSF2-3' UTR-3' Poly(A);
5' Cap-5' UTR-αPDL1-(GGGGS)₃-Antigen-(GS)₃-CSF2-3' UTR-3' Poly(A);
5' Cap-5' UTR-A-C-Antigen-3' UTR-3' Poly(A);
5' Cap-5' UTR-αPDL1-Pan-Antigen-3' UTR-3' Poly(A);
5' Cap-5' UTR-A-Linker-C-Linker-Antigen-3' UTR-3' Poly(A);
5' Cap-5' UTR-αPDL1-Linker-Pan-Linker-Antigen-3' UTR-3' Poly(A);
5' Cap-5' UTR-αPDL1-(GGGGS)₃-Pan-(GS)₃-Antigen-3' UTR-3' Poly(A);
5' Cap-5' UTR-A-Antigen-D-3' UTR-3' Poly(A);
5' Cap-5' UTR-αPDL1-Antigen-Fc-3' UTR-3' Poly(A);
5' Cap-5' UTR-A-Linker-Antigen-Linker-D-3' UTR-3' Poly(A);
5' Cap-5' UTR-αPDL1-Linker-Antigen-Linker-Fc-3' UTR-3' Poly(A);
5' Cap-5' UTR-αPDL1-(GGGGS)₃-Antigen-(G)₃-Fc-3' UTR-3' Poly(A);
5' Cap-5' UTR-B-C-Antigen-3' UTR-3' Poly(A);
5' Cap-5' UTR-CSF2-Pan-Antigen-3' UTR-3' Poly(A);
5' Cap-5' UTR-B-Linker-C-Linker-Antigen-3' UTR-3' Poly(A);
5' Cap-5' UTR-CSF2-Linker-Pan-Linker-Antigen-3' UTR-3' Poly(A);
5' Cap-5' UTR-CSF2-(GGGGS)₃-Pan-(GS)₃-Antigen-3' UTR-3' Poly(A);
5' Cap-5' UTR-B-Antigen-D-3' UTR-3' Poly(A);
5' Cap-5' UTR-CSF2-Antigen-Fc-3' UTR-3' Poly(A);
5' Cap-5' UTR-B-Linker-Antigen-Linker-D-3' UTR-3' Poly(A);
5' Cap-5' UTR-CSF2-Linker-Antigen-Linker-Fc-3' UTR-3' Poly(A);
5' Cap-5' UTR-CSF2-(GGGGS)₃-Antigen-(G)₃-Fc-3' UTR-3' Poly(A);
5' Cap-5' UTR-Antigen-D-B-3' UTR-3' Poly(A);
5' Cap-5' UTR-Antigen-Fc-CSF2-3' UTR-3' Poly(A);
5' Cap-5' UTR-Antigen-Linker-D-Linker-B-3' UTR-3' Poly(A);
5' Cap-5' UTR-Antigen-Linker-Fc-Linker-CSF2-3' UTR-3' Poly(A);
5' Cap-5' UTR-Antigen-(G)₃-Fc-(GS)₃-CSF2-3' UTR-3' Poly(A);
5' Cap-5' UTR-C-Antigen-D-3' UTR-3' Poly(A);
5' Cap-5' UTR-Pan-Antigen-Fc-3' UTR-3' Poly(A);
5' Cap-5' UTR-C-Linker-Antigen-Linker-D-3' UTR-3' Poly(A);
5' Cap-5' UTR-Pan-Linker-Antigen-Linker-Fc-3' UTR-3' Poly(A);
5' Cap-5' UTR-Pan-(GS)₃-Antigen-(G)₃-Fc-3' UTR-3' Poly(A);
5' Cap-5' UTR-A-C-Antigen-B-3' UTR-3' Poly(A);
5' Cap-5' UTR-αPDL1-Pan-Antigen-CSF2-3' UTR-3' Poly(A);
5' Cap-5' UTR-A-Linker-C-Linker-Antigen-Linker-B-3' UTR-3' Poly(A);
5' Cap-5' UTR-αPDL1-Linker-Pan-Linker-Antigen-Linker-CSF2-3' UTR-3' Poly(A);
5' Cap-5' UTR-αPDL1-(GGGGS)₃-Pan-(GS)₃-Antigen-(GS)₃-CSF2-3' UTR-3' Poly(A);
5' Cap-5' UTR-A-Antigen-D-B-3' UTR-3' Poly(A);
5' Cap-5' UTR-αPDL1-Antigen-Fc-CSF2-3' UTR-3' Poly(A);
5' Cap-5' UTR-A-Linker-Antigen-Linker-D-Linker-B-3' UTR-3' Poly(A);
5' Cap-5' UTR-αPDL1-Linker-Antigen-Linker-Fc-Linker-CSF2-3' UTR-3' Poly(A);
5' Cap-5' UTR-αPDL1-(GGGGS)₃-Antigen-(G)₃-Fc-(GS)₃-CSF2-3' UTR-3' Poly(A);
5' Cap-5' UTR-A-C-Antigen-D-3' UTR-3' Poly(A);
5' Cap-5' UTR-αPDL1-Pan-Antigen-Fc-3' UTR-3' Poly(A);
5' Cap-5' UTR-PD1-Pan-Antigen-Fc-3' UTR-3' Poly(A);
5' Cap-5' UTR-CLEC9A binding peptide-Pan-Antigen-Fc-3' UTR-3' Poly(A);
5' Cap-5' UTR-aDEC205-Pan-Antigen-Fc-3' UTR-3' Poly(A);
5' Cap-5' UTR-A-Linker-C-Linker-Antigen-Linker-D-3' UTR-3' Poly(A);
5' Cap-5' UTR-αPDL1-Linker-Pan-Linker-Antigen-Linker-Fc-3' UTR-3' Poly(A);
5' Cap-5' UTR-αPDL1-(GGGGS)₃-Pan-(GS)₃-Antigen-(G)₃-Fc-3' UTR-3' Poly(A);
5' Cap-5' UTR-PD1-Linker-Pan-Linker-Antigen-Linker-Fc-3' UTR-3' Poly(A);
5' Cap-5' UTR-PD1-(GGGGS)₃-Pan-(GS)₃-Antigen-(G)₃-Fc-3' UTR-3' Poly(A);
5' Cap-5' UTR-CLEC9A binding peptide-Linker-Pan-Linker-Antigen-Linker-Fc-3' UTR-3' Poly(A);
5' Cap-5'UTR-CLEC9A binding peptide-(GGGGS)₃-Pan-(GS)₃-Antigen-(G)₃-Fc-3' UTR-3' Poly(A);
5' Cap-5' UTR-αDEC205-Linker-Pan-Linker-Antigen-Linker-Fc-3' UTR-3' Poly(A);
5' Cap-5' UTR-αDEC205-(GGGGS)₃-Pan-(GS)₃-Antigen-(G)₃-Fc-3' UTR-3' Poly(A);
5' Cap-5' UTR-B-C-Antigen-D-3' UTR-3' Poly(A);
5' Cap-5' UTR-IL2-Pan-Antigen-Fc-3' UTR-3' Poly(A);
5' Cap-5' UTR-IL12-Pan-Antigen-Fc-3' UTR-3' Poly(A);
5' Cap-5' UTR-IL21-Pan-Antigen-Fc-3' UTR-3' Poly(A);
5' Cap-5' UTR-CSF2-Pan-Antigen-Fc-3' UTR-3' Poly(A);
5' Cap-5' UTR-IFNα-Pan-Antigen-Fc-3' UTR-3' Poly(A);
5' Cap-5' UTR-B-Linker-C-Linker-Antigen-Linker-D-3' UTR-3' Poly(A);
5' Cap-5' UTR-IL2-Linker-Pan-Linker-Antigen-Linker-Fc-3' UTR-3' Poly(A);
5' Cap-5' UTR-IL2-(GGGGS)₃-Pan-(GS)₃-Antigen-(G)₃-Fc-3' UTR-3' Poly(A);
5' Cap-5' UTR-IL12-Linker-Pan-Linker-Antigen-Linker-Fc-3' UTR-3' Poly(A);
5' Cap-5' UTR-IL12-(GGGGS)₃-Pan-(GS)₃-Antigen-(G)₃-Fc-3' UTR-3' Poly(A);
5' Cap-5' UTR-IL21-Linker-Pan-Linker-Antigen-Linker-Fc-3' UTR-3' Poly(A);
5' Cap-5' UTR-IL21-(GGGGS)₃-Pan-(GS)₃-Antigen-(G)₃-Fc-3' UTR-3' Poly(A);
5' Cap-5' UTR-CSF2-Linker-Pan-Linker-Antigen-Linker-Fc-3' UTR-3' Poly(A);
5' Cap-5' UTR-CSF2-(GGGGS)₃-Pan-(GS)₃-Antigen-(G)₃-Fc-3' UTR-3' Poly(A);
5' Cap-5' UTR-IFNα-Linker-Pan-Linker-Antigen-Linker-Fc-3' UTR-3' Poly(A);
5' Cap-5' UTR-IFNα-(GGGGS)₃-Pan-(GS)₃-Antigen-(G)₃-Fc-3' UTR-3' Poly(A);
5' Cap-5' UTR-C-Antigen-D-B-3' UTR-3' Poly(A);
5' Cap-5' UTR-Pan-Antigen-Fc-CSF2-3' UTR-3' Poly(A);
5' Cap-5' UTR-C-Linker-Antigen-Linker-D-Linker-B-3' UTR-3' Poly(A);
5' Cap-5' UTR-Pan-(GS)₃-Antigen-(G)₃-Fc-(GS)₃-CSF2-3' UTR-3' Poly(A);
5' Cap-5' UTR-A-C-Antigen-D-B-3' UTR-3' Poly(A);
5' Cap-5' UTR-αPDL1-Pan-Antigen-Fc-CSF2-3' UTR-3' Poly(A);
5' Cap-5' UTR-CLEC9A binding peptide-Pan-Antigen-Fc-CSF2-3' UTR-3' Poly(A);
5' Cap-5' UTR-αPDL1-Pan-Antigen-Fc-IFNα-3' UTR-3' Poly(A);
5' Cap-5' UTR-A-Linker-C-Linker-Antigen-Linker-D-Linker-B-3' UTR-3' Poly(A);
5' Cap-5' UTR-αPDL1-Linker-Pan-Linker-Antigen-Linker-Fc-Linker-CSF2-3' UTR-3' Poly(A);
5' Cap-5' UTR-αPDL1-(GGGGS)₃-Pan-(GS)₃-Antigen-(G)₃-Fc-(GS)₃-CSF2-3' UTR-3' Poly(A);
5' Cap-5' UTR-CLEC9A binding peptide-Linker-Pan-Linker-Antigen-Linker-Fc-Linker-CSF2-3' UTR-3' Poly(A);
5' Cap-5' UTR-CLEC9A binding peptide-(GGGGS)₃-Pan-(GS)₃-Antigen-(G)₃-Fc-(GS)₃-CSF2-3' UTR-3' Poly(A);
5' Cap-5' UTR-αPDL1-Linker-Pan-Linker-Antigen-Linker-Fc-Linker-IFNα-3' UTR-3' Poly(A);
5' Cap-5' UTR-αPDL1-(GGGGS)₃-Pan-(GS)₃-Antigen-(G)₃-Fc-(GS)₃-IFNα-3' UTR-3' Poly(A);
5' Cap-5' UTR-A-B- C-Antigen-D-3' UTR-3' Poly(A);
5' Cap-5' UTR-αPDL1-CSF2-Pan-Antigen-Fc-3' UTR-3' Poly(A);
5' Cap-5' UTR-A-Linker-B-Linker- C-Linker-Antigen-Linker-D-3' UTR-3' Poly(A);
5' Cap-5' UTR-αPDL1-Linker-CSF2-Linker-Pan-Linker-Antigen-Linker-Fc-3' UTR-3' Poly(A); and
5' Cap-5' UTR-αPDL1-(GGGGS)₃-CSF2-(GS)₃-Pan-(GS)₃-Antigen-(G)₃-Fc-3' UTR-3' Poly(A).

The above-mentioned structural domain expressing an antigen and an immune cell targeting molecule in the open reading frame of mRNA can be arranged and combined in any form. The domains expressing the antigen and the immune cell targeting molecules A, B, C, and D in the open reading frame can be arranged and combined in any form.

In some embodiments, the open reading frame in the mRNA is an open reading frame for expressing a fusion protein formed by an immune cell targeting molecule and an antigen. There is a schematic diagram of arrangement and combination according to the order of A-antigen, B-antigen, and C-antigen, respectively. An antigen and an immune cell targeting molecule A can be arranged and combined in any form. An antigen and an immune cell targeting molecule B can be arranged and combined in any form. An antigen and an immune cell targeting molecule C can be arranged and combined in any form.

In some embodiments, the open reading frame in the mRNA is an open reading frame for expressing a fusion protein formed by two immune cell targeting molecules and an antigen. There is a schematic diagram of arrangement and combination according to the order of A-B-antigen, A-C-antigen, A-D-antigen, B-C-antigen, B-D-antigen, and C-D-antigen, respectively. An antigen and immune cell targeting molecules A and B can be arranged and combined in any form. An antigen and immune cell targeting molecules A and C can be arranged and combined in any form. An antigen and immune cell targeting molecules A and D can be arranged and combined in any form. An antigens and immune cell targeting molecules B and C can be arranged and combined in any form. An antigen and immune cell targeting molecules B and D can be arranged and combined in any form. An antigen and immune cell targeting molecules C and D can be arranged and combined in any form.

In some embodiments, the open reading frame in the mRNA is an open reading frame for expressing a fusion protein formed by three immune cell targeting molecules and an antigen. There is a schematic diagram of arrangement and combination according to the order of A-B-C-antigen, A-B-D-antigen, A-C-D-antigen, and B-C-D-antigen, respectively. An antigen and immune cell targeting molecules A, B, and C can be arranged and combined in any form. An antigen and immune cell targeting molecules A, B, and D can be arranged and combined in any form. An antigen and immune cell targeting molecules A, C, and D can be arranged and combined in any form. An antigen and immune cell targeting molecules B, C, and D can be arranged and combined in any form.

In some embodiments, the open reading frame in the mRNA is an open reading frame for expressing a fusion protein formed by four immune cell targeting molecules and an antigen. There is a schematic diagram of arrangement and combination in the order of A-B-C-D-antigen. An antigen and immune cell targeting molecules A, B, C, and D can be arranged and combined in any form.

In some embodiments, the antigen is the S protein of SARS-Cov-2 or a fragment thereof. Preferably, the S protein is a pre-fusion stable S protein. In some embodiments, the pre-fusion stable S protein comprises a double proline (S2P) mutation or a six proline (S6P) mutation.

In some embodiments, the antigen is an RBD domain of the S protein of SARS-Cov-2.

In the second aspect, the present application provides a composition comprising the mRNA of the first aspect.

In some embodiments, the composition further comprises a pharmaceutically acceptable carrier. The pharmaceutically acceptable carrier can be a lipid nanoparticle (LNP), a polymer material or an inorganic nanoparticle. For example, the pharmaceutically acceptable carrier can also be cationic lipoplex (LPX), lipid polyplex (LPP), polymer nanoparticle (PNP), inorganic nanoparticle (INP), cationic nanoemulsion (CNE) and the like.

Preferably, the lipid nanoparticle (LNP) can be used for an mRNA vaccine for an infectious disease or an mRNA tumor vaccine. Preferably, the cationic lipoplex (LPX) can be used for an mRNA tumor vaccine or an mRNA vaccine for an infectious disease.

In a third aspect, the present application provides a method for preventing or treating an infection with a pathogenic microorganism or a tumor, wherein the method comprises a step of administering the mRNA of the first aspect or the composition of the second aspect to a subject.

The subject is a human or an animal. The animal includes, but not limited to, cow, sheep, cat, canine, horse, rabbit, monkey, mouse, rat, alpaca, camel, and the like.

In some embodiments, the subject is an immunocompromised human or animal.

In some embodiments, the subject suffers from a chronic lung disease, such as chronic obstructive pulmonary disease or asthma.

In some embodiments, the patient suffers from an underlying disease, such as heart disease, diabetes, or lung disease.

In a fourth aspect, the present application provides a use of the mRNA of the first aspect or the composition of the second aspect in the preparation of a medicament for preventing or treating a disease related to pathogenic microorganism infection or tumor in a subject.

The subject is a human or an animal. The animal includes, but not limited to, cow, sheep, cat, canine, horse, rabbit, monkey, mouse, rat, alpaca, camel, chicken, duck, geese, and the like.

In some embodiments, the subject is an immunocompromised human or animal.

In some embodiments, the subject has a chronic lung disease, such as chronic obstructive pulmonary disease or asthma.

In some embodiments, the patient has an underlying disease, such as heart disease, diabetes, or lung disease.

The mRNA can be delivered to an immune cell, but also to other cells such as a muscle cell or an epithelial cell. The proteins translated by these cells can be secreted and targeted to an immune cell.

The mRNA provided by the present application is capable of expressing a secreted fusion protein or a membrane fusion protein. In some embodiments, the secreted fusion protein expressed by the mRNA can be efficiently targeted to the surface of an immune cell because it contains an immune cell-targeting molecule.

Therefore, the mRNA vaccine according to the present application can enhance immune effect, enhance protection rate, reduce mRNA vaccination dose, enlarge mRNA vaccine production capacity, and reduce mRNA vaccine toxic side effects.

The design of antigen fusion expression in the mRNA vaccine according to the present application can also be applied to other types of vaccines, including but not limited to a nucleic acid vaccine (such as a DNA vaccine and a circular RNA vaccine, etc.), a viral vector vaccine (such as an adenovirus vector vaccine and an influenza virus vector vaccine, etc.), a recombinant protein vaccine and a nanoparticle vaccine, etc.

### TERMs:

### Flanking region: untranslated region (UTR)

An untranslated region (UTR) is capable of being transcribed but not translated. 5' UTR starts from a transcription start site but not include the start codon, while 3' UTR follows the stop codon until the transcription termination signal. UTR affects transcriptional regulation, mRNA stability, and affects translation efficiency. 5' UTR is usually selected from a naturally occurring stable/highly expressed sequence, or engineered from it. 3' UTR is usually related to mRNA stability, and similar to the 5' UTR, it is selected from a naturally occurring sequence, or is modified to some extent.

5' Cap is a very important structure for an mRNA vaccine. It is usually combined with a eukaryotic translation initiation factor to initiate translation. Capping can help mRNA be smoothly transported out of a nucleus. In addition, 5' Cap can also protect mRNA from being degraded by an exonuclease and can also promote the circularization of mRNA to form space and structure, thereby enhancing the stability. Usually, use a capping analog to realize co-transcriptional capping, or use a capping enzyme alone to realize post-transcriptional capping.

3' UTR tail is a PolyA sequence, which is similar to the cap structure. The PolyA tail also protects mRNA from being degraded by an exonuclease. PolyA also participates in translation and regulation processes, thereby increasing stability, extending half-life *in vivo,* and improving mRNA translation efficiency. PolyA-binding protein (PABP) can be connected to the 5' cap through translation initiation factors (such as eIF4G and eIF4E) to form a closed-loop structure and cooperate to participate in the regulation of mRNA stability and translation activity.

Drug delivery has always been a major technical hurdle for the development of mRNA drugs. Most eukaryotic cells actively take up naked mRNA through endocytosis, but this is extremely inefficient, with typically only 1 in 10,000 molecules passing through the cell membrane. After mRNA enters the host, it first faces the degradation by nucleases in the surrounding tissue fluid or blood, and also needs to pass through the negatively charged phospholipid bimolecular biofilm. Therefore, a formulation system of an mRNA drug needs to protect mRNA from nuclease degradation, while also needs to facilitate its uptake by cells. Up to now, the delivery system of mRNA drugs has undergone three generations of technical evolution. From the first-generation delivery system represented by protamine, cationic polymer PEI, and cationic liposome that appeared in the 1990s, to the second-generation delivery system represented by degradable and ionizable polymers that appeared in the late 1990s, and then to the third-generation delivery system represented by ionizable lipid nanoparticles LNP in recent years. Although LNPs currently represent the state-of-the-art RNA delivery technology, the mechanism by which LNPs deliver RNA is not yet fully understood, but it is generally believed that LNPs bind to cell membranes through non-covalent affinity and are endocytosed through a mechanism mediated by clathrin. In cells, mRNA escapes endocytic vesicles and is released into the cytoplasm to express the target protein. However, LNP can also be excreted out of the cell through the opposite exocytosis, which may reduce delivery efficiency of the drug.

The cancer may be prostate cancer, non-small cell lung cancer, small cell lung cancer, renal cell carcinoma, brain cancer, melanoma, acute myeloid leukemia, pancreatic cancer, colorectal cancer, squamous cell carcinoma of the head and neck, squamous cell carcinoma of the skin, adenoid cystic carcinoma, glioblastoma, breast cancer, mesothelioma, ovarian cancer, glioma, bladder cancer, liver cancer, bone cancer, bone marrow cancer, stomach cancer, thyroid cancer, lymphoma, Cervical cancer, endometrial cancer, laryngeal cancer, acute lymphoblastic leukemia, etc.

In some embodiments, the antigen is a tumor antigen, and the tumor antigen includes, but not limited to, 5T4, AIM2, AKAP4 2, Art-4, AuraA1 (AURKA), Aura B1 (AURKB), BAGE, BCAN, B-cyclin, BSG, CCND1, CD133, CDC45L, CDCA1 (TTK), CEA, CHI3L2 (chitinase 3-like 2), CSPG4, EpCAM4, Epha2, EPHX1, Ezh2, FABP7, Fosl1 (Fra-1), GAGE, Galt-3, G250(CA9), gBK, glast, GnT-V, gp100, HB-EGF, HER2, HNPRL, HO-1, hTERT, IGF2BP3, IL13-Ra2, IMP-3, IQGAP1, ITGAV, KIF1C, KIF20A, KIF21B, KIFC3, KK-LC-1, LAGE-1, Lck, LRRC8A, MAGE-1(MAGEA1), MAGE-2(MAGEA2B), MAGE-3, MAGE-4, MAGE-6, MAGE-10, MAGE-12, MAGE-C1(CT7), MAGE-C2, MAGE-C3, Mart-1, MELK, MRP3, MUC1, NAPSA, NLGN4X, Nrcam, NY-ESO-1(CTAG1B), NY-SAR-35, OFA/iLRP, PCNA, PIK3R1, Prame, PRKDC, PTH-rP, PTPRZ1, PTTG1 2, PRKDC, RAN, RGS1, RGS5, RHAMM(RHAMM-3R), RPL19, Sart-1, Sart-2, Sart-3, SEC61G, SGT-1, SOX2, Sox10, Sox11, SP17, SPANX-B, SQSTM1, S.S.X-2, STAT1, STAT3, Survivin, TARA, TNC, Trag-3, TRP-1, TRP2, Tyrosinase, URLC10(LY6K), Ube2V, WT1, XAGE-1b (GAGED2a), YKL-40 (CHI3L1), ACRBP, SCP-1, S.S.X-1, S.S.X-4, NY-TLU-57, CAIX, Brachyury, NY-BR-1, ErbB, Mesothelin, EGFRvIII, IL-13Ra2, MSLN, GPC3, FR, PSMA, GD2, L1-CAM, VEGFR1, VEGFR2, KOC1, OFA, SL-701, mutant P53, DEPDC1, MPHOSPH1, ONT-10, GD2L, GD3L, TF, PAP, BRCA1 DLC1, XPO1, HIF1A, ADAM2, CALR3, SAGE1, SCP-1, ppMAPkkk, WHSC, mutant Ras, COX1, COX2, FOXP3, IDO1, IDO2, TDO, PDL1, PDL2 and PGE2.

In some embodiments, the antigen includes, but not limited to, antigens associated with any tumor/cancer, such as lung cancer (MTFR2 D326Y, CHTF18 L769V, MYADM R30W, HERC1 P3278S, FAM3C K193E, CSMD1G3446E, SLC26A7 R117Q, PGAP1 Y903F, HELB P987S, ANKRD K603T); melanoma (TMEM48F169L, TKT R438W, SEC24A P469L, AKAP13 Q285K, EXOC8 Q656P, PABPC1 R520Q, MRPS5P59L, ABCC2 S1342F, SEC23A P52L, SYTL4 S363F, MAP3K9 E689K, AKAP6 M1482I, RPBMP42L, HCAPG2 P333L, H3F3C T4I, GABPA E161K, SEPT2 Q125R, SRPX P55L, WDR46 T300I, PRDX3 P101L, HELZ2 D614N, GCN1L1 P769L, AFMID A52V, PLSCR4 R247C, CENPL P79L, TPX2H458Y, SEC22C H218Y, POLA2 L420F, SLC24A5 mut); mesothelioma (NOTCH2 G703D, PDE4DIPL288M, BAP1 V523fs, ATP10B E210K, NSD1 K2482T); glioma/glioblastoma (IDH1 R132H, POLE L424V); breast cancer (mPALB2, mROBO3, mZDHHC16, mPTPRS, RBPJ H204L); cholangiocarcinoma (ERBB2IPE805G); and cervical cancer (MAPK1 E322K, PIK3CA E545K, PIK3CA E542K, EP300 D1399N, ERBB2S310F, ERBB3 V104M, KRAS G12D).

The disease related to a pathogenic microorganism includes, but not limited to: Acquired Immunodeficiency Syndrome (AIDS) (Human Immunodeficiency Virus (HIV)); Argentine Teagan fever (Junin virus); Astrovirus infection (Astrovirus Viridae); BK virus infection (BK virus); Bolivian hemorrhagic fever (Machupo virus); Brazilian hemorrhagic fever (Sabiá virus); chickenpox (varicella zoster virus (VZV)); Chikungunya fever (Chikungunya) (alphavirus), Colorado tick fever (CTF) (Colorado tick fever virus (CTFV)); common cold, acute viral nasopharyngitis, acute rhinitis (usually rhinovirus and coronavirus); cytomegalovirus infection (cytomegalovirus); dengue fever (DEN-1, DEN-2, DEN-3 and DEN-4) and other flaviviruses, including but not limited to West Nile virus (West Nile fever), yellow fever virus (Yellow fever); Zika virus (Zika fever) and tick-borne encephalitis virus; Ebola hemorrhagic fever (Ebola virus (EBOV)); enterovirus infection (Enterovirus species); erythema infectious disease (fifth disease) (parvovirus B19); exanthemsubitum (sixth disease) (human herpesvirus 6 (HHV-6) and human herpesvirus 7 (HHV-7) ); Hand, Foot, and Mouth disease (HFMD) (enteroviruses, mainly Coxsackie virus A and enterovirus 71 (EV71)); Hantavirus pulmonary syndrome (HPS) (Sin Nombre virus); hepatitis A (hepatitis A virus); hepatitis B (hepatitis B virus); hepatitis C (hepatitis C virus); hepatitis D (hepatitis D virus); hepatitis E (hepatitis E virus); herpesvirus (herpes simplex virus 1 and 2 (HSV-1 and HSV-2)); human bocavirus infection (human bocavirus (HBoV)); human metapneumovirus infection (human interstitial pneumovirus (hMPV)); Human papillomavirus (HPV) infection (human papillomavirus (HPV)); human parainfluenza virus infection (human parainfluenza virus (HPIV)); Epstein-Barr virus infectious mononucleosis (Mono) (Epstein-Barr virus (EBV)); human influenza viruses (influenza A, including but not limited to H1N1, H2N2, H3N2, H5N1, H7N9, influenza B and other members of the Orthomyxoviridae family); Lassa fever (Lassa arenavirus); lymphocytic choriomeningitis (lymphocytic choriomeningitis virus (LCMV)); Marburg hemorrhagic fever (MHF) (Marburg virus); measles (measles virus); Middle East respiratory syndrome (MERS) (MERS-CoV); Molluscum contagiosum (MC) (Molluscum contagiosum virus (MCV)); Monkeypox (Monkeypox virus); Mumps (Mumps virus); Norovirus (children and infants) (Norovirus); Poliomyelitis (poliomyelitis virus); Progressive multifocal leukoencephalopathy (JC virus); Rabies (Rabies virus); Respiratory Syncytial Virus infection (Respiratory Syncytial Virus (RSV)); Rhinovirus infection (Rhinovirus); Rift Valley fever (RVF) (Rift Valley fever virus); Rotavirus infection (Rotavirus); Rubella (Rubella virus); Herpes zoster (Shingles) (varicella-zoster virus (VZV)); Smallpox (Variola) (variola major or minor); subacute sclerosing panencephalitis (measles virus); Venezuelan equine encephalitis (Venezuelan equine encephalitis virus); Venezuelan hemorrhagic fever (Guararito virus); and Viral pneumonia.

The present application shows that:
(1) The immunogenicity of a free RBD mRNA vaccine is weak, but its immunogenicity is improved when the Fc part is added to an RBD. When a Pan part is added to an RBD-Fc, its immunogenicity is greatly improved. When a cytokine IL12 or CSF2 is added to a Pan-RBD-Fc, its immunogenicity is further improved. When a PD1 or aPDL1 antibody capable of binding to an immune cell surface protein PDL1 is added to a Pan-RBD-Fc, its immunogenicity is further improved;
(2) The immunogenicity of a free RBD mRNA vaccine is weak, but the antibody titer can be increased after being immunized by simply mixing with Pan, and the antibody titer can be further increased after being immunized by fusion expression with Pan;
(3) The antibody titer is not increased after being immunized by simply mixing RBD with αPDL1-Fc, while the antibody titer can be increased after being immunized by fusion expression RBD and αPDL1-Fc. The antibody titer can be increased when adding Fc on the basis of RBD, and the antibody titer can be further increased when adding Pan on the basis of RBD-Fc;
(4) The immunogenicity of a free HA2 mRNA vaccine is weak, but its antibody titer can be increased after being immunized by simply mixing with Pan, and the antibody titer can be further increased after being immunized by fusion expression with Pan. At the same time, the antibody titer can be increased when adding Fc part on the basis of HA2, and the antibody titer can be further increased when adding Pan on the basis of RBD-Fc;
(5) The antibody titer is not increased after being immunized by simply mixing HA2 with αPDL1-Fc, while the antibody titer can be increased after being immunized by fusion expression HA2 and αPDL1-Fc; and the antibody titer can be further increased when adding Pan on the basis of αPDL1-HA2-Fc;
(6) The antibody titer is increased than that of H2A when adding IL12 on the basis of HA2-Fc, and the antibody titer can be further increased when adding Pan on the basis of IL12-HA2-Fc.

### DESCRIPTION OF THE DRAWINGS

FIG. 1 shows a Urea-PAGE electrophoresis identification map of coronavirus SARS-CoV-2 spike protein RBD-related mRNA.
FIG. 2 shows an antibody response against coronavirus SARS-CoV-2 spike protein RBD induced by an immune cell-targeted mRNA vaccine.
FIG. 3 shows a Urea-PAGE electrophoresis identification map of coronavirus SARS-CoV-2 spike protein RBD-related mRNA vaccine.
FIG. 4 shows that an immune cell-targeted coronavirus SARS-CoV-2 spike protein RBD-related mRNA vaccine can elicit a stronger antibody response in mice than a simple RBD mRNA vaccine.
FIG. 5 shows that an immune cell-targeted coronavirus SARS-CoV-2 spike protein RBD-related mRNA vaccine can elicit a stronger antibody response in mice than a simple RBD mRNA vaccine.
FIG. 6 shows a Urea-PAGE electrophoresis identification map of influenza virus Influenza A virus hemagglutinin protein HA-related mRNA vaccine.
FIG. 7 shows that an immune cell-targeted influenza virus Influenza A virus hemagglutinin protein HA-related mRNA vaccine can elicit a stronger antibody response in mice than a simple HA mRNA vaccine.

### BEST MODE

What is described below is the preferred embodiments of the present application, and the protection of the present application is not limited to the following preferred embodiments. It should be pointed out that for those skilled in the art, some modifications and improvements made on the basis of this inventive concept all belong to the protection scope of the present application. The reagents used were not indicated by the manufacturer but were commercially available conventional products.

### SEQUENCEs:

**Start-SP-Human IFNA2-(GGGGS)₃-RBD-Stop** 
AΨGGAGACAGACACACΨCCΨGCΨAΨGGGΨACΨGCΨGCΨCΨGGGΨΨCCAGGΨΨCCACΨGGΨG
ACCGΨACGΨGΨGAΨCΨGCCΨCAGACΨCAΨΨCCCΨGGGΨAGΨAGGAGAACCCΨGAΨGCΨGCΨGG
CACAAAΨGAGAAAAΨCΨCACΨGΨΨAGΨΨGCCΨGAAGGAΨCGACACGACΨΨGGGΨΨCCCC
AAGAAGAGΨΨΨGGCAACCAAΨΨCCAGAAAGCAGAGACAAΨCCCΨGΨCΨΨGCACGAGAΨGAΨCC
AGCAAAΨCΨΨΨAACCΨGΨΨΨCΨACCAAAGAΨΨCCΨCCGCCGCAΨGGGAΨGAGACCCΨGCΨCGA
CAAGΨΨΨAΨACΨGAACΨGΨAΨCAGCAGCΨGAACGAΨΨGGAGGCGΨGCGΨGAΨΨCAGGGCGΨ
ΨGGGGΨΨACCGAAACCCCΨCΨGAΨGAAGGAAGACΨCΨAΨAΨΨGGCΨGΨΨAGGAAGΨAΨΨΨCA
GAGAAΨCACCΨΨGΨAΨΨΨGAAAGAGAAGAAGΨACAGΨCCAΨGΨGCΨΨGGGAGGΨΨGΨCCGGGC
CGAAAΨCAΨGAGAAGΨΨΨCAGCΨΨGΨCAACΨAACCΨGCAGGAAAGCCΨCAGGAGCAAGGAAΨΨ
CGAAGGCGGΨGGAGGCAGCGGAGGCGGΨGGCAGCGGΨGGCGGAGGCAGCΨCΨAGAAGGGΨGCA
GCCCACCGAGAGCAΨCGΨGAGGΨΨCCCCAACAΨCACCAACCΨGΨGCCCCΨΨCGGCGAGGΨGΨΨC
AACGCCACCAGGΨΨCGCCAGCGΨGΨACGCCΨGGAACAGGAAGAGGAΨCAGCAACΨGCGΨGGCCG
ACΨACAGCGΨGCΨGΨACAACAGCGCCAGCΨΨCAGCACCΨΨCAAGΨGCΨACGGCGΨGAGCCCCAC
CAAGCΨGAACGACCΨGΨGCΨΨCACCAACGΨGΨACGCCGACAGCΨΨCGΨGAΨCAGGGGCGACGAG
 
GΨGAGGCAGAΨCGCCCCGGCCAGACCGGCAAGAΨCGCCGACΨACAACΨACAAGCΨGCCCGACG
ACΨΨCACCGGCΨGCGΨGAΨCGCCΨGGAACAGCAACAACCΨGGACAGCAAGGΨGGGCGGCAACΨA
CAACΨACCΨGΨACAGGCΨGΨΨCAGGAAGAGCAACCΨGAAGCCCΨΨCGAGAGGGACAΨCAGCACC
GAGAΨCΨACCAGGCCGGCAGCACCCCCΨGCAACGGCGΨGGAGGGCΨΨCAACΨGCΨACΨΨCCCCC
ΨGCAGAGCΨACGGCΨΨCCAGCCCACCAACGGCGΨGGGCΨACCAGCCCΨACAGGGΨGGΨGGΨGCΨ
GAGCΨΨCGAGCΨGCΨGCACGCCCCCGCCACCGΨGΨGCGGCCCCAAGAAGAGCACCAACCΨGGΨG
AAGAACAAGΨGCGΨGAACΨΨΨCΨCGAGΨAG
**Start-SP-Human CSF2-(GGGGS)₃-RBD-Stop** 
AΨGGAGACAGACACACΨCCΨGCΨAΨGGGΨACΨGCΨGCΨCΨGGGΨΨCCAGGΨΨCCACΨGGΨG
ACCGΨACGGCACCΨGCΨAGAΨCACCAΨCΨCCΨΨCAACACAGCCΨΨGGGAACACGΨΨAACGCCAΨ
ΨCAGGAGGCΨCGGCGCΨΨGCΨGAAΨCΨGAGCAGGGAΨACAGCCGCAGAGAΨGAACGAAACAGΨ
GGAAGΨGAΨΨAGΨGAAAΨGΨΨΨGACCΨΨCAAGAGCCAACAΨGCCΨGCAGACCAGACΨΨGAGCΨ
GΨACAAGCAGGGGCΨGAGGGGAΨCΨCΨΨACAAAACΨGAAAGGGCCACΨΨACCAΨGAΨGGCCΨC
ACACΨAΨAAACAGCACΨGCCCΨCCAACACCΨGAGACGΨCCΨGΨGCΨACΨCAGAΨΨAΨCACΨΨΨC
GAGAGCΨΨΨAAAGAAAAΨCΨGAAGGACΨΨΨCΨCCΨGGΨGAΨCCCCΨΨCGAΨΨGCΨGGGAGCCΨ
GΨCCAGGAGΨΨCGAAGGCGGΨGGAGGCAGCGGAGGCGGΨGGCAGCGGΨGGCGGAGGCAGCΨCΨ
AGAAGGGΨGCAGCCCACCGAGAGCAΨCGΨGAGGΨΨCCCCAACAΨCACCAACCΨGΨGCCCCΨΨCG
GCGAGGΨGΨΨCAACGCCACCAGGΨΨCGCCAGCGΨGΨACGCCΨGGAACAGGAAGAGGAΨCAGCAA
CΨGCGΨGGCCGACΨACAGCGΨGCΨGΨACAACAGCGCCAGCΨΨCAGCACCΨΨCAAGΨGCΨACGGC
GΨGAGCCCCACCAAGCΨGAACGACCΨGΨGCΨΨCACCAACGΨGΨACGCCGACAGCΨΨCGΨGAΨCA
GGGGCGACGAGGΨGAGGCAGAΨCGCCCCCGGCCAGACCGGCAAGAΨCGCCGACΨACAACΨACAA
GCΨGCCCGACGACΨΨCACCGGCΨGCGΨGAΨCGCCΨGGAACAGCAACAACCΨGGACAGCAAGGΨG
GGCGGCAACΨACAACΨACCΨGΨACAGGCΨGΨΨCAGGAAGAGCAACCΨGAAGCCCΨΨCGAGAGGG
ACAΨCAGCACCGAGAΨCΨACCAGGCCGGCAGCACCCCCΨGCAACGGCGΨGGAGGGCΨΨCAACΨG
CΨACΨΨCCCCCΨGCAGAGCΨACGGCΨΨCCAGCCCACCAACGGCGΨGGGCΨACCAGCCCΨACAGG
GΨGGΨGGΨGCΨGAGCΨΨCGAGCΨGCΨGCACGCCCCCGCCACCGΨGΨGCGGCCCCAAGAAGAGCA
CCAACCΨGGΨGAAGAACAAGΨGCGΨGAACΨΨΨCΨCGAGΨAG
**Start-SP-Human IL12-(GGGGS)₃-RBD-Stop** 
AΨGGAGACAGACACACΨCCΨGCΨAΨGGGΨACΨGCΨGCΨCΨGGGΨΨCCAGGΨΨCCACΨGGΨG
ACCGΨACGAΨΨGGGAACΨGAAGAAAGAΨGΨΨACGΨGGΨCGAACΨGGACΨGGΨAΨCCCGAΨG
CGCCΨGGCGAGAΨGGΨAGΨΨCΨGACCΨGΨGACACΨCCCGAAGAAGAΨGGGAΨCACΨΨGGACAΨ
ΨGGAΨCAGΨCAAGCGAAGΨGCΨΨGGGAGΨGGCAAAACAΨΨGACAAΨACAGGΨCAAGGAAΨΨG
GGGACGCΨGGACAGΨACACAΨGCCACAAGGGCGGΨGAGGΨGCΨGΨCACACΨCACΨGCΨGΨΨGC
ΨGCAΨAAAAGGAAGACGGAAΨCΨGGΨCΨACAGACAΨΨCΨGAAGGAΨCAGAAAGAACCΨAAGA
ACAAAACCΨΨCCΨGCGAΨGCGAGGCΨAAGAACΨAΨΨCAGGGCGGΨΨCACGΨGΨΨGGΨGGΨΨGA
CΨACΨAΨΨΨCCACCGAΨCΨGACCΨΨCΨCΨGΨCAAAGCAGCAGAGGCΨCAΨCAGAΨCCCCAAGG
CGΨGACCΨGΨGGCGCAGCΨACCCΨGΨCCGCCGAAAGGGΨCAGAGGAGACAACAAGGAGΨACGAA
ΨAΨAGCGΨGGAGΨGΨCAGGAGGACAGCGCAΨGΨCCΨGCCGCGGAAGAAAGΨΨGCCAAΨΨGAG
GΨGAΨGGΨΨGACGCΨGΨΨCACAAACΨΨAAGΨACGAGAACΨACACCΨCΨAGCΨΨCΨΨΨAΨCAGA
 
GACAΨCAΨΨAAGCCCGACCCCCAAAAAΨCΨGCAGCΨCAAGCCGCΨCAAGAACAGCAGACAGG
ΨCGAGGΨΨCΨΨGGGAGΨACCCCGACACAΨGGΨCΨACΨCCACACΨCΨΨAΨΨΨAGΨCΨCACAΨ
ΨΨΨGCGΨGCAGGΨGCAGGGΨAAGAGΨAAGCGGAGAAAAGGAΨAGAGΨGΨΨACCGACAAAA
CCΨCAGCΨACAGΨGAΨAΨGΨAGAAAGAAΨGCΨAGCAΨCAGΨGΨGCGCGCCCAAGACCGCΨACΨ
ACAGΨΨCΨΨCCΨGGΨCΨGAGΨGGGCGΨCCGΨGCCΨΨGCAGCGGAGGCGGAGGCΨCΨGGAGGAG
GCGGCΨCΨGGCGGAGGCGGAΨCΨCGGAACCΨΨCCΨGΨGGCΨACΨCCΨGAΨCCΨGGCAΨGΨΨCC
ΨΨGΨCΨGCACCACAGCCAGAACΨΨGCΨGCGGGCAGΨGΨCΨAAΨAΨGCΨGCAGAAAGCCAGGCA
GACCCΨGGAGΨΨΨACCCAΨGΨACCAGCGAAGAGAΨΨGAΨCAΨGAGGAΨAΨAACCAAGGACAA
GACΨAGCACCGΨGGAGGCAΨGCCΨGCCACΨGGAGΨΨGACCAAGAACGAAAGCΨGΨCΨCAACΨCC
AGAGAAACΨΨCCΨΨCAΨCACAAACGGΨΨCCΨGCCΨGGCAAGΨAGAAAGACCAGCΨΨAΨGAΨG
GCGCΨGΨGCCΨGΨCΨΨCCAΨCΨAΨGAGGACCΨCAAGAΨGΨACCAGGΨCGAGΨΨAAGACΨAΨG
AACGCCAAGCΨCCΨGAΨGGACCCAAAGCGCCAGAΨΨΨΨΨGGAΨCAGAAΨAΨGΨΨGGCCGΨC
AΨCGAΨGAAΨΨGAΨGCAGGCCCΨΨAACΨΨAAΨAGΨGAGACΨGΨGCCCCAGAAAΨCAAGΨCΨG
GAGGAGCCΨGACΨΨCΨAΨAAGACAAAGAΨCAAGCΨGΨGΨAΨΨΨGCΨΨCACGCAΨΨAGGAΨC
CGCGCΨGΨAACCAΨAGACCGAGΨCAΨGΨCΨΨACCΨGAAΨGCΨAGCΨΨCGAAGGCGGΨGGAGGC
AGCGGAGGCGGΨGGCAGCGGΨGGCGGAGGCAGCΨCΨAGAAGGGΨGCAGCCCACCGAGAGCAΨCG
ΨGAGGΨΨCCCCAACAΨCACCAACCΨGΨGCCCCΨΨCGGCGAGGΨGΨΨCAACGCCACCAGGΨΨCGC
CAGCGΨGΨACGCCΨGGAACAGGAAGAGGAΨCAGCAACΨGCGΨGGCCGACΨACAGCGΨGCΨGΨAC
AACAGCGCCAGCΨΨCAGCACCΨΨCAAGΨGCΨACGGCGΨGAGCCCCACCAAGCΨGAACGACCΨGΨ
GCΨΨCACCAACGΨGΨACGCCGACAGCΨΨCGΨGAΨCAGGGGCGACGAGGΨGAGGCAGAΨCGCCCC
CGGCCAGACCGGCAAGAΨCGCCGACΨACAACΨACAAGCΨGCCCGACGACΨΨCACCGGCΨGCGΨG
AΨCGCCΨGGAACAGCAACAACCΨGGACAGCAAGGΨGGGCGGCAACΨACAACΨACCΨGΨACAGGC
ΨGΨΨCAGGAAGAGCAACCΨGAAGCCCΨΨCGAGAGGGACAΨCAGCACCGAGAΨCΨACCAGGCCGG
CAGCACCCCCΨGCAACGGCGΨGGAGGGCΨΨCAACΨGCΨACΨΨCCCCCΨGCAGAGCΨACGGCΨΨC
CAGCCCACCAACGGCGΨGGGCΨACCAGCCCΨACAGGGΨGGΨGGΨGCΨGAGCΨΨCGAGCΨGCΨGC
ACGCCCCCGCCACCGΨGΨGCGGCCCCAAGAAGAGCACCAACCΨGGΨGAAGAACAAGΨGCGΨGAA
CΨΨCΨCGAGΨAG
**Start-SP-Human IL21-(GGGGS)₃-RBD-Stop** 
AΨGGAGACAGACACACΨCCΨGCΨAΨGGGΨACΨGCΨGCΨCΨGGGΨΨCCAGGΨΨCCACΨGGΨG
ACCGΨACGCACAAGΨCAAGCΨCACAAGGGCAGGACCGGCACAΨGAΨCAGAAΨGAGACAGΨΨGA
ΨCGACAΨAGΨΨGACCAACΨCAAGAACΨACGΨGAACGACCΨCGΨGCCCGAGΨΨCΨGCCCGCCCC
AGAAGACGΨGGAGACGAACΨGΨGAGΨGGΨCCGCAΨΨCΨCCΨGCΨΨCCAGAAGGCΨCAACΨCAA
GAGΨGCCAAΨACCGGGAAΨAAΨGAACGGAΨCAΨCAACGΨGAGCAΨΨAAGAAGCΨGAAACGGAA
ACCΨCCCΨCCACCAAΨGCCGGGAGACGGCAGAAACAΨCGCΨΨGACCΨGΨCCAΨCAΨGΨGAΨCΨ
ΨACGAAAAGAAGCCΨCCCAAGGAAΨΨΨΨGGAACGGΨΨAAGΨCΨΨGCΨCCAGAAGAΨGAΨΨ
CACCAACACCΨGΨCCAGCCGGACCCACGGCAGCGAGGAΨAGCΨΨCGAAGGCGGΨGGAGGCAGCG
GAGGCGGΨGGCAGCGGΨGGCGGAGGCAGCΨCΨAGAAGGGΨGCAGCCCACCGAGAGCAΨCGΨGA
GGΨΨCCCCAACAΨCACCAACCΨGΨGCCCCΨΨCGGCGAGGΨGΨΨCAACGCCACCAGGΨΨCGCCAG
CGΨGΨACGCCΨGGAACAGGAAGAGGAΨCAGCAACΨGCGΨGGCCGACΨACAGCGΨGCΨGΨACAAC
 
AGCGCCAGCΨΨCAGCACCΨΨCAAGΨGCΨACGGCGΨGAGCCCACCAAGCΨGAACGACCΨGΨGCΨ
ΨCACCAACGΨGΨACGCCGACAGCΨΨCGΨGAΨCAGGGCGACGAGGΨGAGGCAGAΨCGCCCCCGG
CCAGACCGGCAAGAΨCGCCGACΨACAACΨACAAGCΨGCCCGACGACΨΨCACCGGCΨGCGΨGAΨC
GCCΨGGAACAGCAACAACCΨGGACAGCAAGGΨGGGCGGCAACΨACAACΨACCΨGΨACAGGCΨGΨ
ΨCAGGAAGAGCAACCΨGAAGCCCΨΨCGAGAGGGACAΨCAGCACCGAGAΨCΨACCAGGCCGGCAG
CACCCCCΨGCAACGGCGΨGGAGGGCΨΨCAACΨGCΨACΨΨCCCCCΨGCAGAGCΨACGGCΨΨCCAG
CCCACCAACGGCGΨGGGCΨACCAGCCCΨACAGGGΨGGΨGGΨGCΨGAGCΨΨCGAGCΨGCΨGCACG
CCCCCGCCACCGΨGΨGCGGCCCCAAGAAGAGCACCAACCΨGGΨGAAGAACAAGΨGCGΨGAACΨΨ
ΨCΨCGAGΨAG
**Start-SP-Human TNR9-(GGGGS)₃-RBD-Stop** 
AΨGGAGACAGACACACΨCCΨGCΨAΨGGGΨACΨGCΨGCΨCΨGGGΨΨCCAGGΨΨCCACΨGGΨG
ACCGΨACGΨΨGCAGGAΨCCΨΨGΨAGCAAΨΨGCCCAGCΨGGCACΨΨΨΨGCGACAACAAΨCGAA
AΨCAAAΨCΨGCΨCACCΨΨGCCCCCCAACΨCΨΨCAGCΨCΨGCAGGCGGCCAGAGGACCΨGCGA
ΨAΨCΨGΨCGCCAGΨGΨAAAGGGΨCΨΨAGAACCAGGAAAGAAΨGΨAGCΨCΨACAΨCΨAAΨGC
CGAGΨGCGAΨΨGΨACCCCGGGGΨΨCAΨΨGCCΨGGGAGCAGGGΨGCΨCAAΨGΨGCGAACAGGA
ΨΨGCAAACAAGGACAGGAACΨGACΨAAGAAAGGAΨGCAAAGACΨGCΨGΨΨCGGAACCΨΨAA
CGAΨCAGAAGCGGGGCAΨCΨGCAGACCΨΨGGACCAACΨGΨΨCΨCΨGGACGGAAAAΨCΨGΨGCΨ
CGΨGAAΨGGGACCAAAGAGAGGGAΨGΨGGΨGΨGΨGGACCCΨCΨCCΨGCΨGACCΨΨAGΨCCΨGG
CGCGΨCΨΨCΨGΨGACΨCCΨCCCGCACCAGCΨAGAGAGCCAGGCCAΨAGΨCCCCAAΨΨCGAAGGC
GGΨGGAGGCAGCGGAGGCGGΨGGCAGCGGΨGGCGGAGGCAGCΨCΨAGAAGGGΨGCAGCCCACC
GAGAGCAΨCGΨGAGGΨΨCCCCAACAΨCACCAACCΨGΨGCCCCΨΨCGGCGAGGΨGΨΨCAACGCCA
CCAGGΨΨCGCCAGCGΨGΨACGCCΨGGAACAGGAAGAGGAΨCAGCAACΨGCGΨGGCCGACΨACAG
CGΨGCΨGΨACAACAGCGCCAGCΨΨCAGCACCΨΨCAAGΨGCΨACGGCGΨGAGCCCCACCAAGCΨG
AACGACCΨGΨGCΨΨCACCAACGΨGΨACGCCGACAGCΨΨCGΨGAΨCAGGGGCGACGAGGΨGAGGC
AGAΨCGCCCCCGGCCAGACCGGCAAGAΨCGCCGACΨACAACΨACAAGCΨGCCCGACGACΨΨCAC
CGGCΨGCGΨGAΨCGCCΨGGAACAGCAACAACCΨGGACAGCAAGGΨGGGCGGCAACΨACAACΨAC
CΨGΨACAGGCΨGΨΨCAGGAAGAGCAACCΨGAAGCCCΨΨCGAGAGGGACAΨCAGCACCGAGAΨCΨ
ACCAGGCCGGCAGCACCCCCΨGCAACGGCGΨGGAGGGCΨΨCAACΨGCΨACΨΨCCCCCΨGCAGAG
CΨACGGCΨΨCCAGCCCACCAACGGCGΨGGGCΨACCAGCCCΨACAGGGΨGGΨGGΨGCΨGAGCΨΨC
GAGCΨGCΨGCACGCCCCCGCCACCGΨGΨGCGGCCCCAAGAAGAGCACCAACCΨGGΨGAAGAACA
AGΨGCGΨGAACΨΨΨCΨCGAGΨAG
**Start-SP-Human TNR4-(GGGGS)₃-RBD-Stop** 
AΨGGAGACAGACACACΨCCΨGCΨAΨGGGΨACΨGCΨGCΨCΨGGGΨΨCCAGGΨΨCCACΨGGΨG
ACCGΨACGCΨGCAΨΨGΨGΨCGGCGAΨACΨΨACCCGΨCΨAACGAΨCGGΨGΨΨGCCACGAGΨGCA
GGCCAGGCAACGGGAΨGGΨΨAGCCGGΨGΨΨCAAGAΨCCCAGAACACAGΨGΨGCCGCCCAΨGΨGG
ACCAGGAΨΨΨAΨAACGACGΨΨGΨΨAGCAGCAAACCAΨGCAAACCCΨGCACAΨGGΨGCAACCΨ
GAGGAGΨGGAAGCGAGCGAAAGCAGCΨCΨGCACAGCCACCCAGGACACCGΨGΨGΨAGGΨGΨCGG
GCAGGCACACAGCCΨCΨΨGACΨCCΨAΨAAACCAGGCGΨΨGAΨΨGΨGCACCGΨGΨCCΨCCAGGΨC
AΨΨΨΨCΨCCAGGCGACAAΨCAGGCCΨGCAAACCCΨGGACΨAACΨGCACCCΨGGCCGGCAAACA
 
ΨACGCΨGCAGCCΨGCCAGCAAΨΨCCAGCGACGCCAΨCΨGCGAAGACAGAGACCCΨCCΨGCΨACA
CAGCCCCAAGAAACCCAGGGΨCCCCCΨGCAAGACCCAΨΨACAGΨGCAGCCAACCGAGGCCΨGGC
CΨAGAACCΨCΨCAGGGGCCAAGCACCAGACCAGΨAGAGGΨGCCGGGCGGΨAGAGCCΨΨCGAAGG
CGGΨGGAGGCAGCGGAGGCGGΨGGCAGCGGΨGGCGGAGGCAGCΨCΨAGAAGGGΨGCAGCCCACC
GAGAGCAΨCGΨGAGGΨΨCCCCAACAΨCACCAACCΨGΨGCCCCΨΨCGGCGAGGΨGΨΨCAACGCCA
CCAGGΨΨCGCCAGCGΨGΨACGCCΨGGAACAGGAAGAGGAΨCAGCAACΨGCGΨGGCCGACΨACAG
CGΨGCΨGΨACAACAGCGCCAGCΨΨCAGCACCΨΨCAAGΨGCΨACGGCGΨGAGCCCCACCAAGCΨG
AACGACCΨGΨGCΨΨCACCAACGΨGΨACGCCGACAGCΨΨCGΨGAΨCAGGGGCGACGAGGΨGAGGC
AGAΨCGCCCCCGGCCAGACCGGCAAGAΨCGCCGACΨACAACΨACAAGCΨGCCCGACGACΨΨCAC
CGGCΨGCGΨGAΨCGCCΨGGAACAGCAACAACCΨGGACAGCAAGGΨGGGCGGCAACΨACAACΨAC
CΨGΨACAGGCΨGΨΨCAGGAAGAGCAACCΨGAAGCCCΨΨCGAGAGGGACAΨCAGCACCGAGAΨCΨ
ACCAGGCCGGCAGCACCCCCΨGCAACGGCGΨGGAGGGCΨΨCAACΨGCΨACΨΨCCCCCΨGCAGAG
CΨACGGCΨΨCCAGCCCACCAACGGCGΨGGGCΨACCAGCCCΨACAGGGΨGGΨGGΨGCΨGAGCΨΨC
GAGCΨGCΨGCACGCCCCCGCCACCGΨGΨGCGGCCCCAAGAAGAGCACCAACCΨGGΨGAAGAACA
AGΨGCGΨGAACΨΨΨCΨCGAGΨAG
**Start-SP-RBD-(GGGGS)₃-Human CD40L-Stop** 
AΨGGAGACAGACACACΨCCΨGCΨAΨGGGΨACΨGCΨGCΨCΨGGGΨΨCCAGGΨΨCCACΨGGΨG
ACCGΨACGAGGGΨGCAGCCCACCGAGAGCAΨCGΨGAGGΨΨCCCCAACAΨCACCAACCΨGΨGCCC
CΨΨCGGCGAGGΨGΨΨCAACGCCACCAGGΨΨCGCCAGCGΨGΨACGCCΨGGAACAGGAAGAGGAΨC
AGCAACΨGCGΨGGCCGACΨACAGCGΨGCΨGΨACAACAGCGCCAGCΨΨCAGCACCΨΨCAAGΨGCΨ
ACGGCGΨGAGCCCCACCAAGCΨGAACGACCΨGΨGCΨΨCACCAACGΨGΨACGCCGACAGCΨΨCGΨ
GAΨCAGGGGCGACGAGGΨGAGGCAGAΨCGCCCCCGGCCAGACCGGCAAGAΨCGCCGACΨACAAC
ΨACAAGCΨGCCCGACGACΨΨCACCGGCΨGCGΨGAΨCGCCΨGGAACAGCAACAACCΨGGACAGCA
AGGΨGGGCGGCAACΨACAACΨACCΨGΨACAGGCΨGΨΨCAGGAAGAGCAACCΨGAAGCCCΨΨCGA
GAGGGACAΨCAGCACCGAGAΨCΨACCAGGCCGGCAGCACCCCCΨGCAACGGCGΨGGAGGGCΨΨC
AACΨGCΨACΨΨCCCCCΨGCAGAGCΨACGGCΨΨCCAGCCCACCAACGGCGΨGGGCΨACCAGCCCΨ
ACAGGGΨGGΨGGΨGCΨGAGCΨΨCGAGCΨGCΨGCACGCCCCCGCCACCGΨGΨGCGGCCCCAAGAA
GAGCACCAACCΨGGΨGAAGAACAAGΨGCGΨGAACΨΨΨΨCGAAGGCGGΨGGAGGCAGCGGAGG
CGGΨGGCAGCGGΨGGCGGAGGCAGCΨCΨAGACACCGAAGGCΨGGAΨAAGAΨΨGAAGACGAAAG
GAACCΨGCACGAGGACΨΨΨGΨΨΨCAΨGAAAACΨAΨCCAGAGAΨGΨAACACGGGCGAAAGGΨC
CCΨGAGCCΨGCΨCAACΨGΨGAGGAAAΨΨAAGΨCΨCAAΨΨCGAGGGΨΨΨGΨCAAGGAΨAΨCAΨ
GCΨCAAΨAAAGAGGAAACGAAGAAAGAAAAΨAGCΨΨGAGAΨGCAGAAAGGΨGACCAGAACCC
ΨCAGAΨΨGCΨGCGCACGΨCAΨAAGCGAGGCCΨCCΨCΨAAAACCACAAGΨGΨGCΨGCAGΨGGGCC
GAAAAAGGCΨACΨAΨACCAΨGΨCCAACAAΨCΨGGΨGACACΨGGAGAAΨGGAAAGCAGCΨCACG
GΨCAAGCGCCAGGGACΨGΨAΨΨAΨAΨCΨACGCACAGGΨΨACCΨΨCΨGΨΨCAAACCGCGAAGCC
ΨCΨAGΨCAGGCGCCAΨΨCAΨΨGCΨΨCCCΨGΨGΨCΨGAAGΨCΨCCΨGGGCGCΨΨCGAGAGAAΨΨ
ΨΨGCΨCCGCGCΨGCCAAΨACGCACΨCAAGCGCCAAACCΨΨGCGGGCAGCAGAGCAΨACACΨΨGG
GAGGAGΨGΨΨΨGAGCΨGCAGCCΨGGCGCΨΨCAGΨGΨΨCGΨGAACGΨGACCGAΨCCΨΨCACAGG
ΨCΨCACACGGGACAGGAΨΨΨACΨAGΨΨΨCGGGCΨΨCΨGAAGΨΨGCΨCGAGΨAG
**Start-SP-Human CSF2-(GGGGS)₃-RBD-(G)₃-Human IgG1 Fc-Stop** 
AΨGGAGACAGACACACΨCCΨGCΨAΨGGGΨACΨGCΨGCΨCΨGGGΨΨCCAGGΨΨCCACΨGGΨG
ACCGΨACGGCACCΨGCΨAGAΨCACCAΨCΨCCΨΨCAACACAGCCΨΨGGGAACACGΨΨAACGCCAΨ
ΨCAGGAGGCΨCGGCGCΨΨGCΨGAAΨCΨGAGCAGGGAΨACAGCCGCAGAGAΨGAACGAAACAGΨ
GGAAGΨGAΨΨAGΨGAAAΨGΨΨΨGACCΨΨCAAGAGCCAACAΨGCCΨGCAGACCAGACΨΨGAGCΨ
GΨACAAGCAGGGGCΨGAGGGAΨCΨCΨΨACAAAACΨGAAAGGGCCACΨΨACCAΨGAΨGGCCΨC
ACACΨAΨAAACAGCACΨGCCCΨCCAACACCΨGAGACGΨCCΨGΨGCΨACΨCAGAΨΨAΨCACΨΨΨC
GAGAGCΨΨΨAAAGAAAAΨCΨGAAGGACΨΨΨCΨCCΨGGΨGAΨCCCCΨΨCGAΨΨGCΨGGGAGCCΨ
GΨCCAGGAGΨΨCGAAGGCGGΨGGAGGCAGCGGAGGCGGΨGGCAGCGGΨGGCGGAGGCAGCΨCΨ
AGAAGGGΨGCAGCCCACCGAGAGCAΨCGΨGAGGΨΨCCCCAACAΨCACCAACCΨGΨGCCCCΨΨCG
GCGAGGΨGΨΨCAACGCCACCAGGΨΨCGCCAGCGΨGΨACGCCΨGGAACAGGAAGAGGAΨCAGCAA
CΨGCGΨGGCCGACΨACAGCGΨGCΨGΨACAACAGCGCCAGCΨΨCAGCACCΨΨCAAGΨGCΨACGGC
GΨGAGCCCCACCAAGCΨGAACGACCΨGΨGCΨΨCACCAACGΨGΨACGCCGACAGCΨΨCGΨGAΨCA
GGGGCGACGAGGΨGAGGCAGAΨCGCCCCGGCCAGACCGGCAAGAΨCGCCGACΨACAACΨACAA
GCΨGCCCGACGACΨΨCACCGGCΨGCGΨGAΨCGCCΨGGAACAGCAACAACCΨGGACAGCAAGGΨG
GGCGGCAACΨACAACΨACCΨGΨACAGGCΨGΨΨCAGGAAGAGCAACCΨGAAGCCCΨΨCGAGAGGG
ACAΨCAGCACCGAGAΨCΨACCAGGCCGGCAGCACCCCCΨGCAACGGCGΨGGAGGGCΨΨCAACΨG
CΨACΨΨCCCCCΨGCAGAGCΨACGGCΨΨCCAGCCCACCAACGGCGΨGGGCΨACCAGCCCΨACAGG
GΨGGΨGGΨGCΨGAGCΨΨCGAGCΨGCΨGCACGCCCCCGCCACCGΨGΨGCGGCCCCAAGAAGAGCA
CCAACCΨGGΨGAAGAACAAGΨGCGΨGAACΨΨΨCΨCGAGGGCGGAGGΨGACAAAACΨCACACAΨ
GCCCACCGΨGCCCAGCACCΨGAACΨCCΨGGGGGGACCGΨCAGΨCΨΨCCΨCΨΨCCCCCCAAAACCC
AAGGACACCCΨCAΨGAΨCΨCCCGGACCCCΨGAGGΨCACAΨGCGΨGGΨGGΨGGACGΨGAGCCACG
AAGACCCΨGAGGΨCAAGΨΨCAACΨGGΨACGΨGGACGGCGΨGGAGGΨGCAΨAAΨGCCAAGACAA
AGCCGCGGGAGGAGCAGΨACAACAGCACGΨACCGΨGΨGGΨCAGCGΨCCΨCACCGΨCCΨGCACCA
GGACΨGGCΨGAAΨGGCAAGGAGΨACAAGΨGCAAGGΨCΨCCAACAAAGCCCΨCCCAGCCCCCAΨC
GAGAAAACCAΨCΨCCAAAGCCAAAGGGCAGCCCCGAGAACCACAGGΨGΨACACCCΨGCCCCCAΨ
CCCGGGAΨGAGCΨGACCAAGAACCAGGΨCAGCCΨGACCΨGCCΨGGΨCAAAGGCΨΨCΨAΨCCCAG
CGACAΨCGCCGΨGGAGΨGGGAGAGCAAΨGGGCAGCCGGAGAACAACΨACAAGACCACGCCΨCCC
GΨGCΨGGACΨCCGACGGCΨCCΨΨCΨΨCCΨCΨACAGCAAGCΨCACCGΨGGACAAGAGCAGGΨGGC
AGCAGGGGAACGΨCΨΨCΨCAΨGCΨCCGΨGAΨGCACGAGGCΨCΨGCACAACCACΨACACGCAGAA
GAGCCΨCΨCCCΨGΨCΨCCGGGΨAAAΨAG
**Start-SP-Pan-(GS)₃-RBD-(G)₃-Human IgG1 Fc-Stop** 
AΨGGAGACAGACACACΨCCΨGCΨAΨGGGΨACΨGCΨGCΨCΨGGGΨΨCCAGGΨΨCCACΨGGΨG
ACCGΨACGGCΨAAAΨΨΨGΨCGCAGCCΨGGACAΨΨGAAAGCAGCCGCΨGGAAGCGGΨAGCGGCA
GCAAGCΨΨAGGGΨGCAGCCCACCGAGAGCAΨCGΨGAGGΨΨCCCCAACAΨCACCAACCΨGΨGCCC
CΨΨCGGCGAGGΨGΨΨCAACGCCACCAGGΨΨCGCCAGCGΨGΨACGCCΨGGAACAGGAAGAGGAΨC
AGCAACΨGCGΨGGCCGACΨACAGCGΨGCΨGΨACAACAGCGCCAGCΨΨCAGCACCΨΨCAAGΨGCΨ
ACGGCGΨGAGCCCCACCAAGCΨGAACGACCΨGΨGCΨΨCACCAACGΨGΨACGCCGACAGCΨΨCGΨ
GAΨCAGGGGCGACGAGGΨGAGGCAGAΨCGCCCCCGGCCAGACCGGCAAGAΨCGCCGACΨACAAC
 
ΨACAAGCΨGCCCGACGACΨΨCACCGGCΨGCGΨGAΨCGCCΨGGAACAGCAACAACCΨGGACAGCA
AGGΨGGGCGGCAACΨACAACΨACCΨGΨACAGGCΨGΨΨCAGGAAGAGCAACCΨGAAGCCCΨΨCGA
GAGGGACAΨCAGCACCGAGAΨCΨACCAGGCCGGCAGCACCCCΨGCAACGGCGΨGGAGGGCΨΨC
AACΨGCΨACΨΨCCCCCΨGCAGAGCΨACGGCΨΨCCAGCCCACCAACGGCGΨGGGCΨACCAGCCCΨ
ACAGGGΨGGΨGGΨGCΨGAGCΨΨCGAGCΨGCΨGCACGCCCCCGCCACCGΨGΨGCGGCCCCAAGAA
GAGCACCAACCΨGGΨGAAGAACAAGΨGCGΨGAACΨΨGAAΨΨCGGCGGAGGΨGACAAAACΨCA
CACAΨGCCCACCGΨGCCCAGCACCΨGAACΨCCΨGGGGGGACCGΨCAGΨCΨΨCCΨCΨΨCCCCCCA
AAACCCAAGGACACCCΨCAΨGAΨCΨCCCGGACCCCΨGAGGΨCACAΨGCGΨGGΨGGΨGGACGΨGA
GCCACGAAGACCCΨGAGGΨCAAGΨΨCAACΨGGΨACGΨGGACGGCGΨGGAGGΨGCAΨAAΨGCCA
AGACAAAGCCGCGGGAGGAGCAGΨACAACAGCACGΨACCGΨGΨGGΨCAGCGΨCCΨCACCGΨCCΨ
GCACCAGGACΨGGCΨGAAΨGGCAAGGAGΨACAAGΨGCAAGGΨCΨCCAACAAAGCCCΨCCCAGCC
CCCAΨCGAGAAAACCAΨCΨCCAAAGCCAAAGGGCAGCCCCGAGAACCACAGGΨGΨACACCCΨGC
CCCCAΨCCCGGGAΨGAGCΨGACCAAGAACCAGGΨCAGCCΨGACCΨGCCΨGGΨCAAAGGCΨΨCΨA
ΨCCCAGCGACAΨCGCCGΨGGAGΨGGGAGAGCAAΨGGGCAGCCGGAGAACAACΨACAAGACCACG
CCΨCCCGΨGCΨGGACΨCCGACGGCΨCCΨΨCΨΨCCΨCΨACAGCAAGCΨCACCGΨGGACAAGAGCA
GGΨGGCAGCAGGGGAACGΨCΨΨCΨCAΨGCΨCCGΨGAΨGCACGAGGCΨCΨGCACAACCACΨACAC
GCAGAAGAGCCΨCΨCCCΨGΨCΨCCGGGΨAAAΨAG
**Start-SP-Human CSF2-(GGGGS)₃-Pan-(GS)₃-RBD-(G)₃-Human IgG1 Fc-Stop** 
AΨGGAGACAGACACACΨCCΨGCΨAΨGGGΨACΨGCΨGCΨCΨGGGΨΨCCAGGΨΨCCACΨGGΨG
ACCGΨACGGCACCΨGCΨAGAΨCACCAΨCΨCCΨΨCAACACAGCCΨΨGGGAACACGΨΨAACGCCAΨ
ΨCAGGAGGCΨCGGCGCΨΨGCΨGAAΨCΨGAGCAGGGAΨACAGCCGCAGAGAΨGAACGAAACAGΨ
GGAAGΨGAΨΨAGΨGAAAΨGΨΨΨGACCΨΨCAAGAGCCAACAΨGCCΨGCAGACCAGACΨΨGAGCΨ
GΨACAAGCAGGGGCΨGAGGGAΨCΨCΨΨACAAAACΨGAAAGGCCACΨΨACCAΨGAΨGGCCΨC
ACACΨAΨAAACAGCACΨGCCCΨCCAACACCΨGAGACGΨCCΨGΨGCΨACΨCAGAΨΨAΨCACΨΨC
GAGAGCΨΨAAAGAAAAΨCΨGAAGGACΨΨCΨCCΨGGΨGAΨCCCCΨΨCGAΨΨGCΨGGGAGCCΨ
GΨCCAGGAGΨΨCGAAGGCGGΨGGAGGCAGCGGAGGCGGΨGGCAGCGGΨGGCGGAGGCAGCGCΨ
AAAΨΨΨGΨCGCAGCCΨGGACAΨΨGAAAGCAGCCGCΨGGAAGCGGΨAGCGGCAGCΨCΨAGAAGG
GΨGCAGCCCACCGAGAGCAΨCGΨGAGGΨΨCCCCAACAΨCACCAACCΨGΨGCCCCΨΨCGGCGAGG
ΨGΨΨCAACGCCACCAGGΨΨCGCCAGCGΨGΨACGCCΨGGAACAGGAAGAGGAΨCAGCAACΨGCGΨ
GGCCGACΨACAGCGΨGCΨGΨACAACAGCGCCAGCΨΨCAGCACCΨΨCAAGΨGCΨACGGCGΨGAGC
CCCACCAAGCΨGAACGACCΨGΨGCΨΨCACCAACGΨGΨACGCCGACAGCΨΨCGΨGAΨCAGGGGCG
ACGAGGΨGAGGCAGAΨCGCCCCCGGCCAGACCGGCAAGAΨCGCCGACΨACAACΨACAAGCΨGCC
CGACGACΨΨCACCGGCΨGCGΨGAΨCGCCΨGGAACAGCAACAACCΨGGACAGCAAGGΨGGGCGGC
AACΨACAACΨACCΨGΨACAGGCΨGΨΨCAGGAAGAGCAACCΨGAAGCCCΨΨCGAGAGGGACAΨCA
GCACCGAGAΨCΨACCAGGCCGGCAGCACCCCCΨGCAACGGCGΨGGAGGGCΨΨCAACΨGCΨACΨΨ
CCCCCΨGCAGAGCΨACGGCΨΨCCAGCCCACCAACGGCGΨGGGCΨACCAGCCCΨACAGGGΨGGΨG
GΨGCΨGAGCΨΨCGAGCΨGCΨGCACGCCCCCGCCACCGΨGΨGCGGCCCCAAGAAGAGCACCAACC
ΨGGΨGAAGAACAAGΨGCGΨGAACΨΨCΨCGAGGGCGGAGGΨGACAAACΨCACACAΨGCCCAC
CGΨGCCCAGCACCΨGAACΨCCΨGGGGGGACCGΨCAGΨCΨΨCCΨCΨΨCCCCCCAAAACCCAAGGA
 
CACCCΨCAΨGAΨCΨCCCGGACCCCΨGAGGΨCACAΨGCGΨGGΨGGΨGGACGΨGAGCCACGAAGAC
CCΨGAGGΨCAAGΨΨCAACΨGGΨACGΨGGACGGCGΨGGAGGΨGCAΨAAΨGCCAAGACAAAGCCG
CGGGAGGAGCAGΨACAACAGCACGΨACCGΨGΨGGΨCAGCGΨCCΨCACCGΨCCΨGCACCAGGACΨ
GGCΨGAAΨGGCAAGGAGΨACAAGΨGCAAGGΨCΨCCAACAAAGCCCΨCCCAGCCCCCAΨCGAGAA
AACCAΨCΨCCAAAGCCAAAGGGCAGCCCCGAGAACCACAGGΨGΨACACCCΨGCCCCCAΨCCCGG
GAΨGAGCΨGACCAAGAACCAGGΨCAGCCΨGACCΨGCCΨGGΨCAAAGGCΨΨCΨAΨCCCAGCGACA
ΨCGCCGΨGGAGΨGGGAGAGCAAΨGGGCAGCCGGAGAACAACΨACAAGACCACGCCΨCCCGΨGCΨ
GGACΨCCGACGGCΨCCΨΨCΨΨCCΨCΨACAGCAAGCΨCACCGΨGGACAAGAGCAGGΨGGCAGCAG
GGGAACGΨCΨΨCΨCAΨGCΨCCGΨGAΨGCACGAGGCΨCΨGCACAACCACΨACACGCAGAAGAGCC
ΨCΨCCCΨGΨCΨCCGGGΨAAAΨAG
Ψ=1-methyl-3'-pseudouridylyl

### Example 1. Design of mRNA vaccine platform

An immune cell-targeted mRNA vaccine consisted of four structural units: 5'cap, 5' UTR, ORF, 3' UTR, and 3'Poly(A). The ORF in an immune cell targeting mRNA vaccine was fused with an immune cell targeting molecule on an antigen, including the following four components: A. an antibody or a polypeptide capable of binding to an immune cell surface protein; B. a cytokine capable of activating an immune cell; C. a Pan epitope (PADRE) capable of activating an immune cell; and D. an immunoglobulin Fc capable of binding an immune cell. The antigen could be arranged and combined with one, two, three or four of these four components in any form to form an open reading frame for expressing fusion protein. The antigen and the immune cell targeting molecule, and the immune cell targeting molecules were connected through a linking fragment.

### Example 2. Construction, production, and identification of mRNA vaccine platform

The construction and production of the vaccine platform are described by taking coronavirus SARS-CoV-2 spike protein RBD as an example.

### 1. Carrier construction

pBluescript II KS (+) was used as a vector, and mRNA vaccine structural unit was constructed downstream of the T7 RNA polymerase promoter by molecular cloning, so as to obtain a plasmid capable of transcribing mRNA. In the plasmid, KOZAK was a Kozak sequence, and SP (signal peptide) was a signal peptide. The antigen and the immune cell targeting molecule, and the immune cell targeting molecules were connected through a linking fragment. The amino acid sequences of linking fragments were as follows: linking fragment 1 (Linker 1) was (G)₃, linking fragment 2 (Linker 2) was (GS)₃, and linking fragment 3 (Linker 3) was (GGGGS)₃.

### RBD:

SacI-5'UTR-XbaI-KOZAK-Start-SP-BsiwI-RBD-XhoI-3'UTR-Poly(A)-NsiI

### RBD-Fc:

SacI-5'UTR-Xbal-KOZAK-Start-SP-BsiwI-RBD-EcoRI-Linker1-Ec-XhoI-3'UTR-Poly(A)-NsiI Pan-RBD-Fc:
SacI-5'UTR-XbaI-KOZAK-Start-SP-BsiwI-Pan-Linker2-HindIII-RBD-EcoRI-Linker1-Fc-XhoI-3'UTR-Po ly(A)-NsiI

### IL4-Pan-RBD-Fc:

SacI-5'UTR-XbaI-KOZAK-Start-SP-BsiwI-IL4-SfuI-Linker3-Pan-Linker2-XbaI-RBD-XhoI-Linker1-Fc-E coRI-3'UTR-Poly(A)-NsiI

### IL10-Pan-RBD-Fc:

SacI-5'UTR-XbaI-KOZAK-Start-SP-BsiwI-IL10-Linker3-SfuI-Pan-Linker2-XbaI-RBD-XhoI-Linker1-Fc-EcoRI-3'UTR-Poly(A)-NsiI

### IL12-Pan-RBD-Fc:

SacI-5'UTR-XbaI-KOZAK-Start-SP-BsiwI-IL12-Linker3-SfuI-Pan-Linker2-XbaI-RBD-XhoI-Linker1-Fc-EcoRI-3'UTR-Poly(A)-NsiI

### CSF2-Pan-RBD-Fc:

SacI-5'UTR-XbaI-KOZAK-Start-SP-BsiwI-CSF2-Linker3-SfuI-Pan-Linker2-XbaI-RBD-XhoI-Linker1-Fc-EcoRI-3'UTR-Poly(A)-NsiI

### PD 1-Pan-RBD-Fc:

SacI-5'UTR-XbaI-KOZAK-Start-SP-BsiwI-PD1-Linker3-SfuI-Pan-Linker2-XbaI-RBD-XhoI-Linker1-Fc-E coRI-3'UTR-Poly(A)-NsiI

### αPDL1-Pan-RBD-Fc:

SacI-5'UTR-XbaI-KOZAK-Start-SP-BsiwI-αPDL1-Linker3-SfuI-Pan-Linker2-XbaI-RBD-XhoI-Linker1-F c-EcoRI-3'UTR-Poly(A)-NsiI

### IFNε-Pan-RBD-Fc:

SacI-5'UTR-XbaI-KOZAK-Start-SP-BsiwI-IFNε-Linker3-SfuI-Pan-Linker2-XbaI-RBD-XhoI-Linker1-Fc-EcoRI-3'UTR-Poly(A)-NsiI

### IFNα4-Pan-RBD-Fc:

SacI-5'UTR-XbaI-KOZAK-Start-SP-BsiwI-IFNα4-Linker3-SfuI-Pan-Linker2-XbaI-RBD-XhoI-Linker1-Fc -EcoRI-3'UTR-Poly(A)-NsiI.

### 2. Preparation of double-stranded DNA template

(1) Endonuclease ScaI and NsiI were used to double digest the constructed plasmid according to the following system: 4 µg of plasmid, 3 µl of ScaI, 3 µl of NsiI, and 6 µl of 10x digestion buffer, added water to make up to 60 µl, and then the system was reacted at 37°C for 1 hour.
(2) 1% of agarose gel was made, and the digested product ran on the gel. The target band was recovered from the gel and eluted with 32 µl of water. The eluted solution contained the double-stranded DNA template.

### 3. In vitro transcription of mRNA

(1) 20 µl of *in vitro* transcription reaction mixture was prepared according to the following system: 7 µl of DNA template, 2 µl of 100 mM DTT, 1 µl of 20 mM ATP, 1 µl of 20 mM CTP, 1 µl of 20 mM GTP, 1 µl of 20 mM N1-UTP, 2 µl of 40 mM ARCA, 2 µl of 10x Transcription buffer (NEB), and 2 µl of T7 RNA polymerase (NEB), to a total of 20 µl. Then, the mixture was incubated at 37°C for 2.5 hours.
(2) The sample was put on ice, added with 1 µl of DNase I, and then reacted at 37°C for 15 minutes.
(3) 0.5 µl of the sample was loaded onto a 1.5% agarose gel, and detected whether there was an mRNA band by electrophoresis.

### 4. Purification of mRNA

The mRNA sample was purified using a RNeasy^{®} MinElute^{®} Cleanup purification kit.
(1) 20 µl of *in vitro* transcription system was added with 80 µl of water, to 100 µl in total.
(2) 350 µl of RLT solution was added and mixed with a pipette.
(3) 250 µl of absolute ethanol was added, mixed well and transferred the solution to a spin column, and centrifuged at 8000 g at room temperature for 15 seconds.
(4) The spin column was transferred to a new 2 ml of centrifuge tube, added with 500 µl of RPE solution, and centrifuged at room temperature for 15 seconds at 8000 g.
(5) 500 µl of 80% ethanol was added, and centrifuged at room temperature for 2 minutes at 8000 g.
(6) The spin column was transferred to a new 2 ml centrifuge tube and centrifuged at the highest speed for 5 minutes.
(7) The spin column was transferred to a 1.5 ml centrifuge tube, 20 µl of water was added to dissolve the mRNA and centrifuged at high speed for 1 minute.

### 5. Identification of concentration and purity of mRNA

100 ng of mRNA was loaded onto a 5% urea-polyacrylamide gel, then, the gel was run at 250V for 2 hours with 0.5×TBE as the electrophoresis buffer, and a gel imager was used to image the integrity and purity of the mRNA.

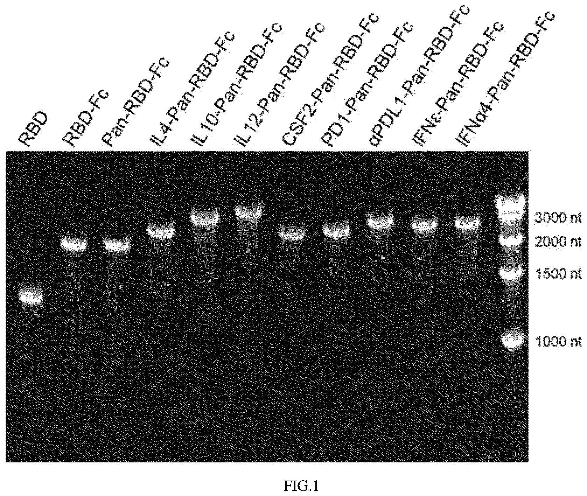

### 6. The Urea-PAGE electrophoresis identification map of the mRNA is shown in FIG. 1.

### Example 3. An immune cell-targeted new coronavirus SARS-CoV-2 spike protein RBD mRNA vaccine can elicit a stronger antibody response in mice than a simple RBD mRNA vaccine

### 1. Materials

C57BL/6 male mice (6-8 weeks) were purchased from Jiangsu Jicui Yaokang Biotechnology Co., Ltd.; horseradish peroxidase (HRP)-labeled goat anti-mouse IgG was purchased from Jiangsu Kangwei Century Biotechnology Co., Ltd.; horseradish peroxidase (HRP)-labeled goat anti-mouse IgG1, IgG2b and IgG2c were purchased from Jiangsu Kangwei Century Biotechnology Co., Ltd.; 96-well ELISA assay plate was purchased from Bioland company; ELISA chromogenic solution was purchased from Shanghai Biyuntian Biotechnology Co., Ltd.; ELISA stop solution was purchased from Beijing Solarbio Science & Technology Co., Ltd.; and microplate reader Multiskan FC was purchased from Thermo Fisher Scientific company.

### 2. Method

(1) Mice were immunized with mRNA vaccine. The mRNA was mixed with the transfection reagent Lipofectamine Messenger MAX with 3 times the mass of the mRNA, and then incubated at room temperature for 5 minutes. Each mouse was inoculated with 0.5 µg of mRNA or the same molar amount of other mRNA, and each mouse was injected intramuscularly with 50 µl. Mice were immunized on Day 0 and Day 21 using two immunization procedures. On Day14 after each immunization, mouse serum was collected by cheek blood collection for antibody detection.
(2) RBD-specific antibodies in serum were detected by ELISA. RBD coating solution (2 µg/ml) was added to the Elisa plate at 100 µl per well and coated overnight at 4°C. 5% blocking solution (5% FBS in PBS) was used to block at 37°C for 1 hour, and PBST was used to wash for 3 times. Serum samples were diluted according to 10-fold gradient, and 100 µl was added to each well into a well-blocked ELISA plate and incubated at 37°C for 1 hour. PBST was used to wash for 3 times, 100 µl of enzyme-labeled secondary antibody (1:5000) was added to each well and incubated at 37°C for 1 hour. PBST was used to wash for 5 times, substrate TMB was added at 100 µl/well, and incubated at room temperature in the dark, waited for substrate color development; 50 µl of ELISA stop solution was added to each well to stop color development, and the plate reading was recorded with a microplate reader at OD450-620.

### 3. Results

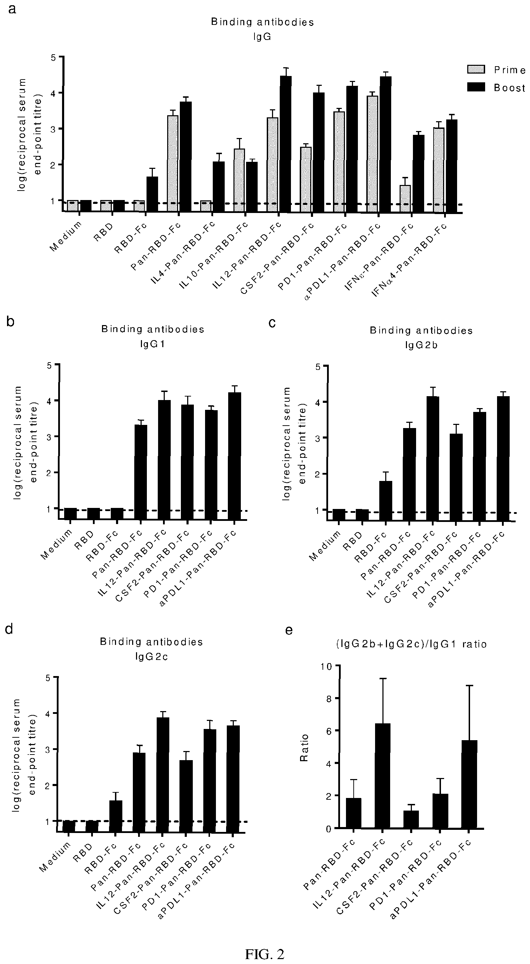

The immunogenicity of the free RBD mRNA vaccine was weak, and the immunogenicity was improved when the Fc part was added on the basis of RBD, and the immunogenicity was extremely improved when the Pan part was added on the basis of RBD-Fc. The immunogenicity was further improved when cytokine IL12 or CSF2 was added on the basis of Pan-RBD-Fc, and the immunogenicity was further improved when PD1 or aPDL1 antibody capable of binding an immune cell surface protein PDL1 was added on the basis of Pan-RBD-Fc, and the antibody level after the second immunization was generally higher than that after the first immunization, as shown in FIG. 2(a). IL12, CSF2, PD1 or aPDL1 induced higher levels of IgG1 representing Th2 immune responses on the basis of Pan-RBD-Fc, as shown in FIG. 2(b). IL12, PD1 or aPDL1 induced higher levels of IgG2b and IgG2c representing Th1 immune responses on the basis of Pan-RBD-Fc, as shown in FIG. 2(c) and FIG. 2(d). The ratio of (IgG2b+IgG2c)/IgG1 showed that Pan-RBD-Fc, CSF2-Pan-RBD-Fc and PD1-Pan-RBD-Fc induced relatively balanced Th1 and Th2 immune responses, while IL12-Pan-RBD-Fc and aPDL1-Pan-RBD-Fc induced a Th1-biased immune response, as shown in FIG. 2(e).

### Example 4. Construction, production and identification of mRNA vaccine related to coronavirus SARS-CoV-2 spike protein RBD

### 1. Carrier construction

pBluescript II KS(+) was used as a vector, and mRNA vaccine structural unit was constructed downstream of the T7 RNA polymerase promoter by molecular cloning, so as to obtain a plasmid capable of transcribing mRNA. In the plasmid, KOZAK was a Kozak sequence, and SP (signal peptide) was a signal peptide. The antigen and the immune cell targeting molecule, and the immune cell targeting molecules were connected through a linking fragment. The amino acid sequences of linking fragments were as follows: linking fragment 1 (Linker 1) was (G)₃, linking fragment 2 (Linker 2) was (GS)₃, and linking fragment 3 (Linker 3) was (GGGGS)₃.

### Pan:

SacI-5'UTR-XbaI-KOZAK-Start-SP-BsiwI-Pan-XhoI-3'UTR-Poly(A)-NsiI

### Pan-RBD:

SacI-5'UTR-XbaI-KOZAK-Start-SP-BsiwI-Pan-Linker2-HindIII-RBD-XhoI-3'UTR-Poly(A)-NsiI αPDL1-Fc:
SacI-5'UTR-XbaI-KOZAK-Start-SP-BsiwI-αPDL1-SfuI-Linker1-Fc-EcoRI-3'UTR-Poly(A)-NsiI αPDL1-RBD-Fc:
SacI-5'UTR-XbaI-KOZAK-Start-SP-BsiwI-αPDL1-SfuI-Einker3-XbaI-RBD-XhoI-Linker1-Fc-EcoRI-3'U TR-Poly(A)-NsiI

### αPDL1-RBD-Fc-IFNα4:

SacI-5'UTR-XbaI-KOZAK-Start-SP-BsiwI-αPDL1-SfuI-Linker3-XbaI-RBD-XhoI-Linker1-Fc-Linker3-IF Na4-EcoRI-3'UTR-Poly(A)-NsiI

### IL12-RBD-Fc:

SacI-5'UTR-XbaI-KOZAK-Start-SP-BsiwI-IL12-SfuI-Linker3-XbaI-RBD-XhoI-Linker1-Fc-EcoRI-3'UTR -Poly(A)-NsiI.

### 2. Preparation of double-stranded DNA template

(1) Endonuclease Sea I and Nsi I were used to double digest the constructed plasmid according to the following system: 4 µg of plasmid, 3 µl of Sea I, 3 µl of Nsi I, and 6 µl of 10x digestion buffer, added water to make up to 60 µl, and then the system was reacted at 37°C for 1 hour.
(2) 1% of agarose gel was made, and the digested product run on the gel. The target band was recovered from the gel and eluted with 32 µl of water. The eluted solution contained the double-stranded DNA template.

### 3. In vitro transcription of mRNA

(1) 20 µl of *in vitro* transcription reaction mixture was prepared according to the following system: 7 µl of DNA template, 2 µl of 100 mM DTT, 1 µl of 20 mM ATP, 1 µl of 20 mM CTP, 1 µl of 20 mM GTP, 1 µl of 20 mM N1-UTP, 2 µl of 40 mM ARCA, 2 µl of 10x Transcription buffer (NEB), and 2 µl of T7 RNA polymerase (NEB), to a total of 20 µl. Then, the mixture was incubated at 37°C for 2.5 hours.
(2) The sample was put on ice, added with 1 µl of DNase I, and then reacted at 37°C for 15 minutes.
(3) 0.5 µl of the sample was loaded onto a 1.5% agarose gel, and detected whether there was an mRNA band by electrophoresis.

### 4. Purification of mRNA

The mRNA sample was purified using a RNeasy^{®} MinElute^{®} Cleanup purification kit.
(1) 20 µl of *in vitro* transcription system was added with 80 µl of water, to 100 µl in total.
(2) 350 µl of RLT solution was added and mixed with a pipette.
(3) 250 µl of absolute ethanol was added, mixed well and transferred the solution to a spin column, and centrifuged at 8000 g at room temperature for 15 seconds.
(4) The spin column was transferred to a new 2 ml of centrifuge tube, added with 500 µl of RPE solution, and centrifuged at room temperature for 15 seconds at 8000 g.
(5) 500 µl of 80% ethanol was added, and centrifuged at room temperature for 2 minutes at 8000 g.
(6) The spin column was transferred to a new 2 ml centrifuge tube and centrifuged at the highest speed for 5 minutes.
(7) The spin column was transferred to a 1.5 ml centrifuge tube, 20 µl of water was added to dissolve the mRNA and centrifuged at high speed for 1 minute.

### 5. Identification of concentration and purity of mRNA

100 ng of mRNA was loaded onto a 5% urea-polyacrylamide gel, then, the gel was run at 250V for 2 hours with 0.5×TBE as the electrophoresis buffer, and a gel imager was used to image the integrity and purity of the mRNA.

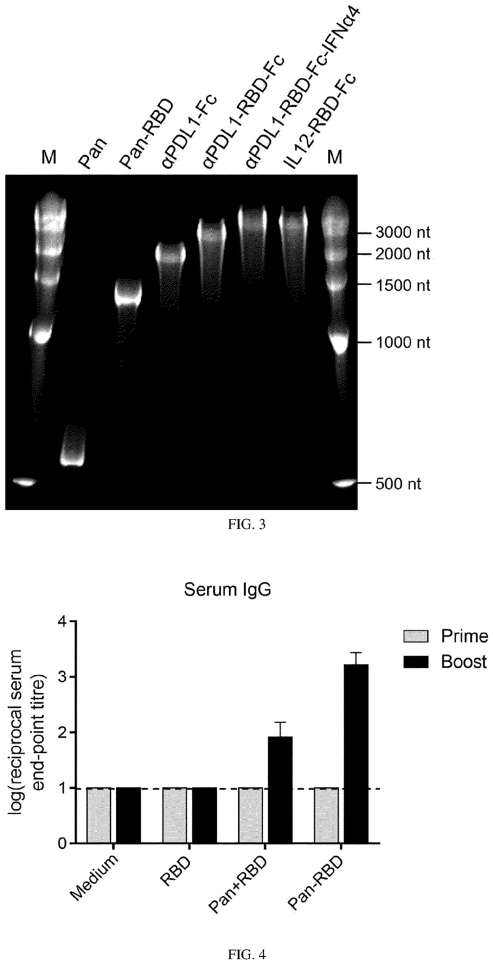

### 6. The Urea-PAGE electrophoresis identification map of the mRNA is shown in FIG. 3.

### Example 5. An immune cell-targeted new coronavirus SARS-CoV-2 spike protein RBD related mRNA vaccine can elicit a stronger antibody response in mice than a simple RBD mRNA vaccine

### 1. Materials

C57BL/6 male mice (6-8 weeks) were purchased from Jiangsu Jicui Yaokang Biotechnology Co., Ltd.; horseradish peroxidase (HRP)-labeled goat anti-mouse IgG was purchased from Jiangsu Kangwei Century Biotechnology Co., Ltd.; 96-well ELISA assay plate was purchased from Bioland company; ELISA chromogenic solution was purchased from Shanghai Biyuntian Biotechnology Co., Ltd.; ELISA stop solution was purchased from Beijing Solarbio Science & Technology Co., Ltd.; and microplate reader Multiskan FC was purchased from Thermo Fisher Scientific company.

### 2. Method

(1) Mice were immunized with mRNA vaccine. The mRNA was mixed with the transfection reagent Lipofectamine Messenger MAX with 3 times the mass of the mRNA, and then incubated at room temperature for 5 minutes. Each mouse was inoculated with 0.5 µg of mRNA or the same molar amount of other mRNA, and each mouse was injected intramuscularly with 50 µl. Mice were immunized on Day 0 and Day 21 using two immunization procedures. On Day14 after each immunization, mouse serum was collected by cheek blood collection for antibody detection.
(2) RBD-specific antibodies in serum were detected by ELISA. RBD coating solution (2 µg/ml) was added to the Elisa plate at 100 µl per well and coated overnight at 4°C. 5% blocking solution (5% FBS in PBS) was used to block at 37°C for 1 hour, and PBST was used to wash for 3 times. Serum samples were diluted according to 10-fold gradient, and 100 µl was added to each well into a well-blocked ELISA plate and incubated at 37°C for 1 hour. PBST was used to wash for 3 times, 100 µl of enzyme-labeled secondary antibody (1:5000) was added to each well and incubated at 37°C for 1 hour. PBST was used to wash for 5 times, substrate TMB was added at 100 µl/well, and incubated at room temperature in the dark, waited for substrate color development; 50 µl of ELISA stop solution was added to each well to stop color development, and the plate reading was recorded with a microplate reader at OD450-620.

### 3. Results

See FIG. 4, the immunogenicity of the free RBD mRNA vaccine was weak, and its antibody titer could be increased after immunized by simply mixing with Pan, and the antibody titer could be further increased after immunized by fusion expression with Pan. These results indicated that the immunogenicity of Pan-RBD was higher than that of simply mixing Pan and RBD.

### Example 6. An immune cell-targeted new coronavirus SARS-CoV-2 spike protein RBD related mRNA vaccine can elicit a stronger antibody response in mice than a simple RBD mRNA vaccine

### 1. Materials

C57BL/6 male mice (6-8 weeks) were purchased from Jiangsu Jicui Yaokang Biotechnology Co., Ltd.; horseradish peroxidase (HRP)-labeled goat anti-mouse IgG was purchased from Jiangsu Kangwei Century Biotechnology Co., Ltd.; 96-well ELISA assay plate was purchased from Bioland company; ELISA chromogenic solution was purchased from Shanghai Biyuntian Biotechnology Co., Ltd.; ELISA stop solution was purchased from Beijing Solarbio Science & Technology Co., Ltd.; and microplate reader Multiskan FC was purchased from Thermo Fisher Scientific company.

### 2. Method

(1) Mice were immunized with mRNA vaccine.
   Four components of SM-102, DSPC, Cholesterol and DMG-PEG2000 were dissolved in ethanol at a molar ratio of 50:10:38.5:1.5, mRNA was dissolved in 20 mM of sodium acetate (pH=4). The mRNA and liposome were mixed according to the mass ratio of 1:40 and the volume ratio of 1:3 to form mRNA/liposome nanoparticles and performed ultrafiltration to remove ethanol and filter sterilization. PBS (pH=7.4) was used to dilute the concentration of mRNA/liposome nanoparticles to 1 µg of mRNA/100 µl, and 100 µl of intramuscular injection was administered to mice for immunization. Mice were immunized on Day 0 and Day 21 using two immunization procedures. On day14 after each immunization, mouse serum was collected by cheek blood collection for antibody detection.
(2) RBD-specific antibodies in serum were detected by ELISA. RBD coating solution (2 µg/ml) was added to the Elisa plate at 100 µl per well and coated overnight at 4°C. 5% blocking solution (5% FBS in PBS) was used to block at 37°C for 1 hour, and PBST was used to wash for 3 times. Serum samples were diluted according to 10-fold gradient, and 100 µl was added to each well into a well-blocked ELISA plate and incubated at 37°C for 1 hour. PBST was used to wash for 3 times, 100 µl of enzyme-labeled secondary antibody (1:5000) was added to each well and incubated at 37°C for 1 hour. PBST was used to wash for 5 times, substrate TMB was added at 100 µl/well, and incubated at room temperature in the dark, waited for substrate color development; 50 µl of ELISA stop solution was added to each well to stop color development, and the plate reading was recorded with a microplate reader at OD450-620.

### 3. Results

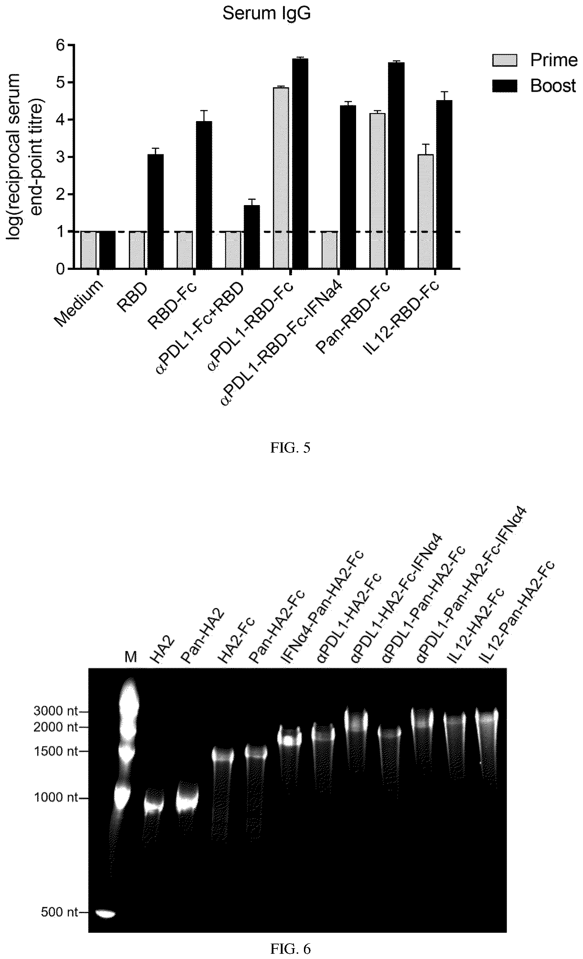

See FIG. 5, the antibody titer was not increased after immunized by simply mixing RBD and αPDL1-Fc, the antibody titer could be increased after immunized by fusion expression of RBD and αPDL1-Fc, while the antibody titer was decreased when IFNα4 was added on the basis of αPDL1-RBD-Fc.The antibody titer could be increased when Fc was added on the basis of RBD, and the antibody titer could be increased when Pan was added on the basis of RBD-Fc, while the antibody titer could not be increased when IL12 was added on the basis of RBD-Fc.

### Example 7. Construction, production and identification of mRNA vaccine related to influenza virus Influenza A virus hemagglutinin protein HA

There are at least 18 different subtypes of HA in influenza A virus. H1 in human influenza virus was selected as the research object. Each HA is cleaved by cellular proteases into HA1 subunit and HA2 subunit. HA1 subunit forming the globular head, while containing the predominant neutralizing epitope, is highly variable between subtypes. HA2 subunit constitutes most of the stalk domain, is fairly conserved among subtypes, and contains few but cross-reactive neutralizing epitopes. The conserved HA2 subunit was selected as the research object.

### 1. Carrier construction

pBluescript II KS(+) was used as a vector, and mRNA vaccine structural unit was constructed downstream of the T7 RNA polymerase promoter by molecular cloning, so as to obtain a plasmid capable of transcribing mRNA. In the plasmid, KOZAK was a Kozak sequence, and SP (signal peptide) was a signal peptide. The antigen and the immune cell targeting molecule, and the immune cell targeting molecules were connected through a linking fragment. The amino acid sequences of linking fragments were as follows: linking fragment 1 (Linker 1) was (G)₃, linking fragment 2 (Linker 2) was (GS)₃, and linking fragment 3 (Linker 3) was (GGGGS)₃.

### HA2:

SacI-5'UTR-XbaI-KOZAK-Start-SP-BsiwI-HA2-EcoRI-3'UTR-Poly(A)-NsiI Pan-HA2:
SacI-5'UTR-XbaI-KOZAK-Start-SP-BsiwI-Pan-Linker2-HindIII-HA2-XhoI-3'UTR-Poly(A)-NsiI HA2-Fc:
SacI-5'UTR-XbaI-KOZAK-Start-SP-BsiwI-HA2-XhoI-Linker1-Fc-EcoRI-3'UTR-Poly(A)-NsiI Pan-HA2-Fc:
SacI-5'UTR-XbaI-KOZAK-Start-SP-BsiwI-Pan-Linker2-HindIII-HA2-EcoRI-Linker1-Fc-XhoI-3'UTR-Pol y(A)-NsiI

### IFNα4-Pan-HA2-Fc:

SacI-5'UTR-XbaI-KOZAK-Start-SP-BsiwI-IFNα4-SfuI-Linker3-Pan-Linker2-XbaI-HA2-XhoI-Linker1-Fc -EcoRI-3'UTR-Poly(A)-NsiI

### αPDL1-HA2-Fc:

SacI-5'UTR-XbaI-KOZAK-Start-SP-BsiwI-αPDL1-SfuI-Linker3-XbaI-HA2-XhoI-Linker1-Fc-EcoRI-3'U TR-Poly(A)-NsiI

### αPDL1-HA2-Fc-IFNα4:

SacI-5'UTR-XbaI-KOZAK-Start-SP-BsiwI-αPDL1 -SfuI-Linker3-XbaI-HA2-XhoI-Linker 1 -Fc-Linker3-IF Nα4-EcoRI-3'UTR-Poly(A)-NsiI

### αPDL1-Pan-HA2-Fc:

SacI-5'UTR-XbaI-KOZAK-Start-SP-BsiwI-αPDL1-SfuI-Linker3-Pan-Linker2-XbaI-HA2-XhoI-Linker1-Fc -EcoRI-3'UTR-Poly(A)-NsiI

### αPDL1-Pan-HA2-Fc-IFNα4:

SacI-5'UTR-XbaI-KOZAK-Start-SP-BsiwI-αPDL1-SfuI-Linker3-Pan-Linker2-XbaI-HA2-XhoI-Linker1-Fc -Linker3-IFNα4-EcoRI-3'UTR-Poly(A)-NsiI

### IL12-HA2-Fc:

SacI-5'UTR-XbaI-KOZAK-Start-SP-BsiwI-IL12-Linker3-XbaI-HA2-XhoI-Linker1-Fc-EcoRI-3'UTR-Poly (A)-Nsil

### IL12-Pan-HA2-Fc:

SacI-5'UTR-XbaI-KOZAK-Start-SP-BsiwI-IL12-SfuI-Linker3-Pan-Linker2-XbaI-HA2-XhoI-Linker1-Fc-E coRI-3'UTR-Poly(A)-NsiI.

### 2. Preparation of double-stranded DNA template

(1) Endonuclease Sea I and Nsi I were used to double digest the constructed plasmid according to the following system: 4 µg of plasmid, 3 µl of Sea I, 3 µl of Nsi I, and 6 µl of 10x digestion buffer, added water to make up to 60 µl, and then the system was reacted at 37°C for 1hour;
(2) 1% of agarose gel was made, and the digested product run on the gel. The target band was recovered from the gel, and eluted with 32 µl of water. The eluted solution contained the double-stranded DNA template.

### 3. In vitro transcription of mRNA

(1) 20 µl of *in vitro* transcription reaction mixture was prepared according to the following system: 7 µl of DNA template, 2 µl of 100 mM DTT, 1 µl of 20 mM ATP, 1 µl of 20 mM CTP, 1 µl of 20 mM GTP, 1 µl of 20 mM N1-UTP, 2 µl of 40 mM ARCA, 2 µl of 10x Transcription buffer (NEB), and 2 µl of T7 RNA polymerase (NEB), to a total of 20 µl. Then, the mixture was reacted at 37°C for 2.5 hours.
(2) The sample was put on ice, added with 1 µl of DNase I, and then reacted at 37°C for 15 minutes.
(3) 0.5 µl of the sample was loaded onto a 1.5% agarose gel, and detected whether there was an mRNA band by electrophoresis.

### 4. Purification of mRNA

The mRNA sample was purified using a RNeasy^{®} MinElute^{®} Cleanup purification kit.
(1) 20 µl of *in vitro* transcription system was added with 80 µl of water, to 100 µl in total.
(2) 350 µl of RLT solution was added and mixed with a pipette.
(3) 250 µl of absolute ethanol was added, mixed well and transferred the solution to a spin column, and centrifuged at 8000 g at room temperature for 15 seconds.
(4) The spin column was transferred to a new 2 ml of centrifuge tube, added with 500 µl of RPE solution, and centrifuged at room temperature for 15 seconds at 8000 g.
(5) 500 µl of 80% ethanol was added, and centrifuged at room temperature for 2 minutes at 8000 g.
(6) The spin column was transferred to a new 2 ml centrifuge tube and centrifuged at the highest speed for 5 minutes.
(7) The spin column was transferred to a 1.5 ml centrifuge tube, 20 µl of water was added to dissolve the mRNA and centrifuged at high speed for 1 minute.

### 5. Identification of concentration and purity of mRNA

100 ng of mRNA was loaded onto a 5% urea-polyacrylamide gel, then, the gel was run at 250V for 2 hours with 0.5×TBE as the electrophoresis buffer, and a gel imager was used to image the integrity and purity of the mRNA.

### 6. The Urea-PAGE electrophoresis identification map of the mRNA is shown in FIG. 6.

### Example 8. An immune cell-targeted influenza virus Influenza A virus hemagglutinin protein HA-related mRNA vaccine can elicit a stronger antibody response in mice than a simple HA mRNA vaccine

### 1. Materials

C57BL/6 male mice (6-8 weeks) were purchased from Jiangsu Jicui Yaokang Biotechnology Co., Ltd.; horseradish peroxidase (HRP)-labeled goat anti-mouse IgG was purchased from Jiangsu Kangwei Century Biotechnology Co., Ltd.; 96-well ELISA assay plate was purchased from Bioland company; ELISA chromogenic solution was purchased from Shanghai Biyuntian Biotechnology Co., Ltd.; ELISA stop solution was purchased from Beijing Solarbio Science & Technology Co., Ltd.; and microplate reader Multiskan FC was purchased from Thermo Fisher Scientific company.

### 2. Method

(1) Four components of SM-102, DSPC, Cholesterol and DMG-PEG2000 were dissolved in ethanol at a molar ratio of 50:10:38.5:1.5, mRNA was dissolved in 20 mM of sodium acetate (pH=4). The mRNA and liposome were mixed according to the mass ratio of 1:40 and the volume ratio of 1:3 to form mRNA/liposome nanoparticles and performed ultrafiltration to remove ethanol and filter sterilization. PBS (pH=7.4) was used to dilute the concentration of mRNA/liposome nanoparticles to 1 µg of mRNA/100 µl, and 100 µl of intramuscular injection was administered to mice for immunization. Mice were immunized on Day 0 and Day 21 using two immunization procedures. On day14 after each immunization, mouse serum was collected by cheek blood collection for antibody detection.
(2) RBD-specific antibodies in serum were detected by ELISA. RBD coating solution (2 µg/ml) was added to the Elisa plate at 100 µl per well and coated overnight at 4°C. 5% blocking solution (5% FBS in PBS) was used to block at 37°C for 1 hour, and PBST was used to wash for 3 times. Serum samples were diluted according to 10-fold gradient, and 100 µl was added to each well into a well-blocked ELISA plate and incubated at 37°C for 1 hour. PBST was used to wash for 3 times, 100 µl of enzyme-labeled secondary antibody (1:5000) was added to each well and incubated at 37°C for 1 hour. PBST was used to wash for 5 times, substrate TMB was added at 100 µl/well, and incubated at room temperature in the dark, waited for substrate color development; 50 µl of ELISA stop solution was added to each well to stop color development, and the plate reading was recorded with a microplate reader at OD450-620.

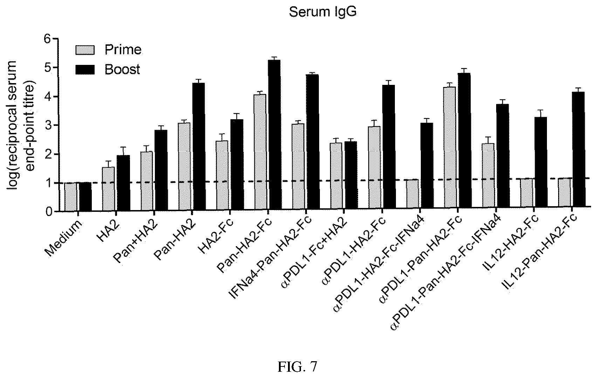

### 3. The results were shown in FIG. 7:

(1) The immunogenicity of the free HA2 mRNA vaccine was weak, and the antibody titer could be increased after immunized by simply mixing with Pan, and the antibody titer could be further increased after immunized by fusion expression with Pan. At the same time, the antibody titer could be increased when Fc part was added on the basis of HA2, and the antibody titer could be further improved when Pan was added on the basis of HA2-Fc, while the antibody titer was decreased when IFNα4 was added on the basis of Pan-HA2-Fc, as shown in FIG. 7. These results indicated that the immunogenicity of Pan-HA2 was higher than that of HA2 and the simple mixture of Pan and HA2, and the immunogenicity of Pan-HA2-Fc was higher than that of HA2, HA2-Fc and IFNα4-Pan-HA2-Fc.
(2) The antibody titer was not increase after immunized by simply mixing HA2 and αPDL1-Fc, and the antibody titer could be increased after immunized by fusion expression of HA2 and αPDL1-Fc, while the antibody titer was decreased when IFNα4 was added on the basis of αPDL1-HA2-Fc. The antibody titer could be increased when Pan was added on the basis of αPDL1-HA2-Fc, while the antibody titer was decreased when IFNα4 was added on the basis of PDL1-Pan-HA2-Fc, as shown in FIG. 7. These results indicated that the immunogenicity of αPDL1-HA2-Fc was higher than that of HA2, the simple mixture of αPDL1-Fc and HA2, HA2-Fc and αPDL1-HA2-Fc-IFNα4. The immunogenicity of αPDL1-Pan-HA2-Fc was higher than that of HA2, HA2-Fc, αPDL1-HA2-Fc and αPDL1-Pan-HA2-Fc-IFNα4.
(3) The antibody titer was not increased when IL12 was added on the basis of HA2-Fc, but it was increased compared to HA2. The antibody titer could be increased when Pan was added on the basis of IL12-HA2-Fc, as shown in the FIG. 7. These results indicated that the immunogenicity of IL12-HA2-Fc was higher than that of HA2. The immunogenicity of IL12-Pan-HA2-Fc was higher than that of HA2, HA2-Fc and IL12-HA2-Fc.

## Claims

1. An mRNA containing an open reading frame (ORF), wherein the open reading frame encodes an antigen and an immune cell targeting molecule.

2. The mRNA according to claim 1, wherein the antigen and the immune cell targeting molecule encoded by the open reading frame are a fusion protein of the antigen and the immune cell targeting molecule.

3. The mRNA according to claim 1, wherein the immune cell targeting molecule is one or more selected from the group consisting of:
A: an antibody or a polypeptide capable of binding to an immune cell surface protein;
B: a cytokine capable of activating an immune cell;
C: Pan epitope (PADRE) capable of activating an immune cell; and
D: an immunoglobulin Fc capable of binding to an immune cell;
preferably, the A is selected from an antibody against CD274 (PDL1), PDCD1LG2 (PDL2), CLEC9A, LY75 (DEC205), CD40, TNFSF9 (4-1BB-L) and/or TNFSF4 (OX4OL), or an active fragment of a ligand thereof;
preferably, the B is selected from interleukin (IL), colony-stimulating factor (CSF) and/or interferon (IFN), or an active fragment thereof, more preferably, the interleukin is selected from IL2, IL12, IL15 and/or IL21 or an active fragment thereof;
preferably, the colony-stimulating factor is selected from CSF1, CSF2 and/or CSF3, or an active fragment thereof;
preferably, the interferon is selected from type I interferon, type II interferon and/or type III interferon, or an active fragment thereof, more preferably, the type I interferon is selected from IFNα, IFNω, IFNε, IFNκ and/or IFNβ, or an active fragment thereof; more preferably, the type II interferon is selected from IFN-γ, or an active fragment thereof; more preferably, the type III interferon is selected from IFN-λ1, IFN-λ2, IFN-λ3 and/or IFN-λ4, or an active fragment thereof;
preferably, the C has an amino acid sequence as shown by AKFVAAWTLKAAA (SEQ ID NO: 1); and
preferably, the D is selected from Fc from IgG, IgM, IgA, IgE or IgD, or a mutant thereof.

4. The mRNA according to any one of claims 1 to 3, wherein the open reading frame encodes a fusion protein of an antigen with A; a fusion protein of an antigen with A and B; a fusion protein of an antigen with A, B and C; a fusion protein of an antigen with A, B, C and D; a fusion protein of an antigen with A and C; a fusion protein of an antigen with A and D; a fusion protein of an antigen with A, C and D; a fusion protein of an antigen with B; a fusion protein of an antigen with B and C; a fusion protein of an antigen with B and D; a fusion protein of an antigen with B, C and D; a fusion protein of an antigen with C; a fusion protein of an antigen with C and D; or a fusion protein of an antigen with D;
the fusion protein is any arrangement of the antigen with A and/or B and/or C and/or D; or
the antigen and A and/or B and/or C and/or D in the fusion protein are located at the C-terminus or N-terminus relatively to each other;
preferably, the fusion protein is a homodimer or a heterodimer,
preferably, the fusion protein is a secreted fusion protein or a membrane fusion protein,
more preferably, the fusion protein is a secreted fusion protein;
the antibody or polypeptide A capable of binding to an immune cell surface protein is selected from an antibody against CD274 (PDL1), PDCD1LG2 (PDL2), CLEC9A, LY75 (DEC205), CD40, TNFSF9 (4-1BB-L), and/or TNFSF4 (OX4OL), or an active fragment of a ligand thereof.

5. The mRNA according to any one of claims 1 to 4, wherein the mRNA further comprises a 5' UTR sequence and a 3' UTR sequence.

6. The mRNA according to any one of claims 1 to 5, wherein the mRNA further comprises a 5' Cap and a 3' Poly(A).

7. The mRNA according to any one of claims 1 to 6, wherein the open reading frame (ORF) further comprises a region encoding a linking fragment,
preferably, the antigen and the immune cell targeting molecule, and/or the immune cell targeting molecules are connected through a linking fragment;
preferably, the linking fragment is a flexible linking fragment, a rigid linking fragment or an *in vivo* cleavage linking fragment, more preferably, an amino acid sequence of the flexible linking fragment is (G)_{N}, (GS)_{N}, (GGS)_{N}, (GGGS)_{N}, or (GGGGS)_{N}.

8. The mRNA according to any one of claims 1 to 7, wherein the mRNA has a structure selected from the group consisting of:
**5' Cap-5' UTR-A-Antigen-3' UTR-3' Poly(A);**
5' Cap-5' UTR-λPDL1-Antigen-3' UTR-3' Poly(A);
5' Cap-5' UTR-CLEC9A binding peptide-Antigen-3' UTR-3' Poly(A);
5' Cap-5' UTR-aDEC205-Antigen-3' UTR-3' Poly(A);
**5' Cap-5' UTR-A-Linker-Antigen-3' UTR-3' Poly(A);**
5' Cap-5' UTR-αPDL1-Linker-Antigen-3' UTR-3' Poly(A);
5' Cap-5' UTR-αPDL1-(GGGGS)₃-Antigen-3' UTR-3' Poly(A);
5' Cap-5' UTR-CLEC9A binding peptide-Linker-Antigen-3' UTR-3' Poly(A);
5' Cap-5' UTR-CLEC9A binding peptide-(GGGGS)₃-Antigen-3' UTR-3' Poly(A);
5' Cap-5' UTR-αDEC205-Linker-Antigen-3' UTR-3' Poly(A);
5' Cap-5' UTR-αDEC205-(GGGGS)₃-Antigen-3' UTR-3' Poly(A);
**5' Cap-5' UTR-Antigen-A-3' UTR-3' Poly(A);**
5' Cap-5' UTR-Antigen-αPDL1-3' UTR-3' Poly(A);
5' Cap-5' UTR-Antigen-CLEC9A binding peptide-3' UTR-3' Poly(A);
5' Cap-5' UTR-Antigen-CD40L-3' UTR-3' Poly(A);
5' Cap-5' UTR-Antigen-4-1BB-3' UTR-3' Poly(A);
5' Cap-5' UTR-Antigen-OX40-3' UTR-3' Poly(A);
**5' Cap-5' UTR-Antigen-Linker-A-3' UTR-3' Poly(A);**
5' Cap-5' UTR-Antigen-Linker-αPDL1-3' UTR-3' Poly(A);
5' Cap-5' UTR-Antigen-(GGGGS)₃-αPDL1-3' UTR-3' Poly(A);
5' Cap-5' UTR-Antigen-Linker-CLEC9A binding peptide-3' UTR-3' Poly(A);
5' Cap-5' UTR-Antigen-(GGGGS)₃-CLEC9A binding peptide-3' UTR-3' Poly(A);
5' Cap-5' UTR-Antigen-Linker-CD40L-3' UTR-3' Poly(A);
5' Cap-5' UTR-Antigen-(GGGGS)₃-CD40L-3' UTR-3' Poly(A);
5' Cap-5' UTR-Antigen-Linker-4-1BB-3' UTR-3' Poly(A);
5' Cap-5' UTR-Antigen-(GGGGS)₃-4-1BB-3' UTR-3' Poly(A);
5' Cap-5' UTR-Antigen-Linker-OX40-3' UTR-3' Poly(A);
5' Cap-5' UTR-Antigen-(GGGGS)₃-OX40-3' UTR-3' Poly(A);
**5' Cap-5' UTR-B-Antigen-3' UTR-3' Poly(A);**
5' Cap-5' UTR-IL2-Antigen-3' UTR-3' Poly(A);
5' Cap-5' UTR-IL12-Antigen-3' UTR-3' Poly(A);
5' Cap-5' UTR-IL15-Antigen-3' UTR-3' Poly(A);
5' Cap-5' UTR-IL21-Antigen-3' UTR-3' Poly(A);
5' Cap-5' UTR-CSF2-Antigen-3' UTR-3' Poly(A);
5' Cap-5' UTR-IFNa-Antigen-3' UTR-3' Poly(A);
**5' Cap-5' UTR-B-Linker-Antigen-3' UTR-3' Poly(A);**
5' Cap-5' UTR-IL2-Linker-Antigen-3' UTR-3' Poly(A);
5' Cap-5' UTR-IL2-(GGGGS)₃-Antigen-3' UTR-3' Poly(A);
5' Cap-5' UTR-IL12-Linker-Antigen-3' UTR-3' Poly(A);
5' Cap-5' UTR-IL12-(GGGGS)₃-Antigen-3' UTR-3' Poly(A);
5' Cap-5' UTR-IL15-Linker-Antigen-3' UTR-3' Poly(A);
5' Cap-5' UTR-IL15-(GGGGS)₃-Antigen-3' UTR-3' Poly(A);
5' Cap-5' UTR-IL21-Linker-Antigen-3' UTR-3' Poly(A);
5' Cap-5' UTR-IL21-(GGGGS)₃-Antigen-3' UTR-3' Poly(A);
5' Cap-5' UTR-CSF2-Linker-Antigen-3' UTR-3' Poly(A);
5' Cap-5' UTR-CSF2-(GGGGS)₃-Antigen-3' UTR-3' Poly(A);
5' Cap-5' UTR-IFNa-Linker-Antigen-3' UTR-3' Poly(A);
5' Cap-5' UTR-IFNα-(GGGGS)₃-Antigen-3' UTR-3' Poly(A);
**5' Cap-5' UTR-Antigen-B-3' UTR-3' Poly(A);**
5' Cap-5' UTR-Antigen-CSF2-3' UTR-3' Poly(A);
**5' Cap-5' UTR-Antigen-Linker-B-3' UTR-3' Poly(A);**
5' Cap-5' UTR-Antigen-Linker-CSF2-3' UTR-3' Poly(A);
5' Cap-5' UTR-Antigen-(GGGGS)₃-CSF2-3' UTR-3' Poly(A);
**5' Cap-5' UTR-C-Antigen-3' UTR-3' Poly(A);**
5' Cap-5' UTR-Pan-Antigen-3' UTR-3' Poly(A);
**5' Cap-5' UTR-C-Linker-Antigen-3' UTR-3' Poly(A);**
5' Cap-5' UTR-Pan-Linker-Antigen-3' UTR-3' Poly(A);
5' Cap-5' UTR-Pan-(GS)₃-Antigen-3' UTR-3' Poly(A);
**5' Cap-5' UTR-Antigen-C-3' UTR-3' Poly(A);**
5' Cap-5' UTR-Antigen-Pan-3' UTR-3' Poly(A);
**5' Cap-5' UTR-Antigen-Linker-C-3' UTR-3' Poly(A);**
5' Cap-5' UTR-Antigen-Linker-Pan-3' UTR-3' Poly(A);
5' Cap-5' UTR-Antigen-(GS)₃-Pan-3' UTR-3' Poly(A);
**5' Cap-5' UTR-A-Antigen-B-3' UTR-3' Poly(A);**
5' Cap-5' UTR-αPDL1-Antigen-CSF2-3' UTR-3' Poly(A);
**5' Cap-5' UTR-A-Linker-Antigen-Linker-B-3' UTR-3' Poly(A);**
5' Cap-5' UTR-αPDL1-Linker-Antigen-Linker-CSF2-3' UTR-3' Poly(A);
5' Cap-5' UTR-αPDL1-(GGGGS)₃-Antigen-(GS)₃-CSF2-3' UTR-3' Poly(A);
**5' Cap-5' UTR-A-C-Antigen-3' UTR-3' Poly(A);**
5' Cap-5' UTR-αPDL1-Pan-Antigen-3' UTR-3' Poly(A);
**5' Cap-5' UTR-A-Linker-C-Linker-Antigen-3' UTR-3' Poly(A);**
5' Cap-5' UTR-αPDL1-Linker-Pan-Linker-Antigen-3' UTR-3' Poly(A);
5' Cap-5' UTR-αPDL1-(GGGGS)₃-Pan-(GS)₃-Antigen-3' UTR-3' Poly(A);
**5' Cap-5' UTR-A-Antigen-D-3' UTR-3' Poly(A);**
5' Cap-5' UTR-αPDL1-Antigen-Fc-3' UTR-3' Poly(A);
**5' Cap-5' UTR-A-Linker-Antigen-Linker-D-3' UTR-3' Poly(A);**
5' Cap-5' UTR-αPDL1-Linker-Antigen-Linker-Fc-3' UTR-3' Poly(A);
5' Cap-5' UTR-αPDL1-(GGGGS)₃-Antigen-(G)₃-Fc-3' UTR-3' Poly(A);
**5' Cap-5' UTR-B-C-Antigen-3' UTR-3' Poly(A);**
5' Cap-5' UTR-CSF2-Pan-Antigen-3' UTR-3' Poly(A);
**5' Cap-5' UTR-B-Linker-C-Linker-Antigen-3' UTR-3' Poly(A);**
5' Cap-5' UTR-CSF2-Linker-Pan-Linker-Antigen-3' UTR-3' Poly(A);
5' Cap-5' UTR-CSF2-(GGGGS)₃-Pan-(GS)₃-Antigen-3' UTR-3' Poly(A);
**5' Cap-5' UTR-B-Antigen-D-3' UTR-3' Poly(A);**
5' Cap-5' UTR-CSF2-Antigen-Fc-3' UTR-3' Poly(A);
**5' Cap-5' UTR-B-Linker-Antigen-Linker-D-3' UTR-3' Poly(A);**
5' Cap-5' UTR-CSF2-Linker-Antigen-Linker-Fc-3' UTR-3' Poly(A);
5' Cap-5' UTR-CSF2-(GGGGS)₃-Antigen-(G)₃-Fc-3' UTR-3' Poly(A);
**5' Cap-5' UTR-Antigen-D-B-3' UTR-3' Poly(A);**
5' Cap-5' UTR-Antigen-Fc-CSF2-3' UTR-3' Poly(A);
**5' Cap-5' UTR-Antigen-Linker-D-Linker-B-3' UTR-3' Poly(A);**
5' Cap-5' UTR-Antigen-Linker-Fc-Linker-CSF2-3' UTR-3' Poly(A);
5' Cap-5' UTR-Antigen-(G)₃-Fc-(GS)₃-CSF2-3' UTR-3' Poly(A);
**5' Cap-5' UTR-C-Antigen-D-3' UTR-3' Poly(A);**
5' Cap-5' UTR-Pan-Antigen-Fc-3' UTR-3' Poly(A);
**5' Cap-5' UTR-C-Linker-Antigen-Linker-D-3' UTR-3' Poly(A);**
5' Cap-5' UTR-Pan-Linker-Antigen-Linker-Fc-3' UTR-3' Poly(A);
5' Cap-5' UTR-Pan-(GS)₃-Antigen-(G)₃-Fc-3' UTR-3' Poly(A);
**5' Cap-5' UTR-A-C-Antigen-B-3' UTR-3' Poly(A);**
5' Cap-5' UTR-αPDL1-Pan-Antigen-CSF2-3' UTR-3' Poly(A);
**5' Cap-5' UTR-A-Linker-C-Linker-Antigen-Linker-B-3' UTR-3' Poly(A);**
5' Cap-5' UTR-αPDL1-Linker-Pan-Linker-Antigen-Linker-CSF2-3' UTR-3' Poly(A);
5' Cap-5' UTR-αPDL1-(GGGGS)₃-Pan-(GS)₃-Antigen-(GS)₃-CSF2-3' UTR-3' Poly(A);
**5' Cap-5' UTR-A-Antigen-D-B-3' UTR-3' Poly(A);**
5' Cap-5' UTR-αPDL1-Antigen-Fc-CSF2-3' UTR-3' Poly(A);
**5' Cap-5' UTR-A-Linker-Antigen-Linker-D-Linker-B-3' UTR-3' Poly(A);**
5' Cap-5' UTR-αPDL1-Linker-Antigen-Linker-Fc-Linker-CSF2-3' UTR-3' Poly(A);
5' Cap-5' UTR-αPDL1-(GGGGS)₃-Antigen-(G)₃-Fc-(GS)₃-CSF2-3' UTR-3' Poly(A);
**5' Cap-5' UTR-A-C-Antigen-D-3' UTR-3' Poly(A);**
5' Cap-5' UTR-αPDL1-Pan-Antigen-Fc-3' UTR-3' Poly(A);
5' Cap-5' UTR-PD1-Pan-Antigen-Fc-3' UTR-3' Poly(A);
5' Cap-5' UTR-CLEC9A binding peptide-Pan-Antigen-Fc-3' UTR-3' Poly(A);
5' Cap-5' UTR-aDEC205-Pan-Antigen-Fc-3' UTR-3' Poly(A);
**5' Cap-5' UTR-A-Linker-C-Linker-Antigen-Linker-D-3' UTR-3' Poly(A);**
5' Cap-5' UTR-αPDL1-Linker-Pan-Linker-Antigen-Linker-Fc-3' UTR-3' Poly(A);
5' Cap-5' UTR-αPDL1-(GGGGS)₃-Pan-(GS)₃-Antigen-(G)₃-Fc-3' UTR-3' Poly(A);
5' Cap-5' UTR-PDI-Linker-Pan-Linker-Antigen-Linker-Fc-3' UTR-3' Poly(A);
5' Cap-5' UTR-PD1-(GGGGS)₃-Pan-(GS)₃-Antigen-(G)₃-Fc-3' UTR-3' Poly(A);
5' Cap-5' UTR-CLEC9A binding peptide-Linker-Pan-Linker-Antigen-Linker-Fc-3' UTR-3' Poly(A);
5' Cap-5' UTR-CLEC9A binding peptide-(GGGGS)₃-Pan-(GS)₃-Antigen-(G)₃-Fc-3' UTR-3' Poly(A);
5' Cap-5' UTR-aDEC205-Linker-Pan-Linker-Antigen-Linker-Fc-3' UTR-3' Poly(A);
5' Cap-5' UTR-αDEC205-(GGGGS)₃-Pan-(GS)₃-Antigen-(G)₃-Fc-3' UTR-3' Poly(A);
**5' Cap-5' UTR-B-C-Antigen-D-3' UTR-3' Poly(A);**
5' Cap-5' UTR-IL2-Pan-Antigen-Fc-3' UTR-3' Poly(A);
5' Cap-5' UTR-IL12-Pan-Antigen-Fc-3' UTR-3' Poly(A);
5' Cap-5' UTR-IL21-Pan-Antigen-Fc-3' UTR-3' Poly(A);
5' Cap-5' UTR-CSF2-Pan-Antigen-Fc-3' UTR-3' Poly(A);
5' Cap-5' UTR-IFNa-Pan-Antigen-Fc-3' UTR-3' Poly(A);
**5' Cap-5' UTR-B-Linker-C-Linker-Antigen-Linker-D-3' UTR-3' Poly(A);**
5' Cap-5' UTR-IL2-Linker-Pan-Linker-Antigen-Linker-Fc-3' UTR-3' Poly(A);
5' Cap-5' UTR-IL2-(GGGGS)₃-Pan-(GS)₃-Antigen-(G)₃-Fc-3' UTR-3' Poly(A);
5' Cap-5' UTR-IL12-Linker-Pan-Linker-Antigen-Linker-Fc-3' UTR-3' Poly(A);
5' Cap-5' UTR-IL12-(GGGGS)₃-Pan-(GS)₃-Antigen-(G)₃-Fc-3' UTR-3' Poly(A);
5' Cap-5' UTR-IL21-Linker-Pan-Linker-Antigen-Linker-Fc-3' UTR-3' Poly(A);
5' Cap-5' UTR-IL21-(GGGGS)₃-Pan-(GS)₃-Antigen-(G)₃-Fc-3' UTR-3' Poly(A);
5' Cap-5' UTR-CSF2-Linker-Pan-Linker-Antigen-Linker-Fc-3' UTR-3' Poly(A);
5' Cap-5' UTR-CSF2-(GGGGS)₃-Pan-(GS)₃-Antigen-(G)₃-Fc-3' UTR-3' Poly(A);
5' Cap-5' UTR-IFNa-Linker-Pan-Linker-Antigen-Linker-Fc-3' UTR-3' Poly(A);
5' Cap-5' UTR-IFNα-(GGGGS)₃-Pan-(GS)₃-Antigen-(G)₃-Fc-3' UTR-3' Poly(A);
**5' Cap-5' UTR-C-Antigen-D-B-3' UTR-3' Poly(A);**
5' Cap-5' UTR-Pan-Antigen-Fc-CSF2-3' UTR-3' Poly(A);
**5' Cap-5' UTR-C-Linker-Antigen-Linker-D-Linker-B-3' UTR-3' Poly(A);**
5' Cap-5' UTR-Pan-(GS)₃-Antigen-(G)₃-Fc-(GS)₃-CSF2-3' UTR-3' Poly(A);
**5' Cap-5' UTR-A-C-Antigen-D-B-3' UTR-3' Poly(A);**
5' Cap-5' UTR-αPDL1-Pan-Antigen-Fc-CSF2-3' UTR-3' Poly(A);
5' Cap-5' UTR-CLEC9A binding peptide-Pan-Antigen-Fc-CSF2-3' UTR-3' Poly(A);
5' Cap-5' UTR-αPDL1-Pan-Antigen-Fc-IFNα-3' UTR-3' Poly(A);
**5' Cap-5' UTR-A-Linker-C-Linker-Antigen-Linker-D-Linker-B-3' UTR-3' Poly(A);**
5' Cap-5' UTR-αPDL1-Linker-Pan-Linker-Antigen-Linker-Fc-Linker-CSF2-3' UTR-3' Poly(A);
5' Cap-5' UTR-αPDL1-(GGGGS)₃-Pan-(GS)₃-Antigen-(G)₃-Fc-(GS)₃-CSF2-3' UTR-3' Poly(A);
5' Cap-5' UTR-CLEC9A binding peptide-Linker-Pan-Linker-Antigen-Linker-Fc-Linker-CSF2-3' UTR-3' Poly(A);
5' Cap-5' UTR-CLEC9A binding peptide-(GGGGS)₃-Pan-(GS)₃-Antigen-(G)₃-Fc-(GS)₃-CSF2-3' UTR-3' Poly(A);
5' Cap-5' UTR-αPDL1-Linker-Pan-Linker-Antigen-Linker-Fc-Linker-IFNα-3' Poly(A);
5' Cap-5' UTR-αPDL1-(GGGGS)₃-Pan-(GS)₃-Antigen-(G)₃-Fc-(GS)₃-IFNα-3'UTR-3' Poly(A);
**5' Cap-5' UTR-A-B- C-Antigen-D-3' UTR-3' Poly(A);**
5' Cap-5' UTR-αPDL1-CSF2-Pan-Antigen-Fc-3' UTR-3' Poly(A);
**5' Cap-5' UTR-A-Linker-B-Linker- C-Linker-Antigen-Linker-D-3' UTR-3' Poly(A);**
5' Cap-5' UTR-αPDL1-Linker-CSF2-Linker-Pan-Linker-Antigen-Linker-Fc-3' UTR-3' Poly(A); and
5' Cap-5' UTR-αPDL1-(GGGGS)₃-CSF2-(GS)₃-Pan-(GS)₃-Antigen-(G)₃-Fc-3' UTR-3' Poly(A).

9. The mRNA according to any one of claims 1 to 8, wherein the antigen is an immunogenic protein or an immunogenic fragment thereof capable of inducing an immune response against a pathogenic microorganism;
preferably, the pathogenic microorganism is SARS-Cov-2, SARS, cytomegalovirus (CMV), herpes virus, RSV, influenza virus, Ebola virus, Epstein-Barr virus (EBV), dengue virus, Zike virus, HIV virus, rabies virus, plasmodium gametophyte, herpes zoster virus (HZV), hepatitis B virus (HBV), hepatitis C virus (HCV), hepatitis D virus (HDV), HPV, *Mycobacterium tuberculosis,* or *Helicobacter pylori.*

10. The mRNA according to claim 9, wherein the antigen is S protein of SARS-Cov-2 or a fragment thereof, preferably, the S protein is a pre-fusion stable S protein, more preferably, the pre-fusion stable S protein comprises a double proline (S2P) mutation; or the antigen is an RBD domain of the S protein of SARS-Cov-2.

11. The mRNA according to any one of claims 1 to 8, wherein the antigen is an immunogenic protein or an immunogenic fragment thereof capable of inducing an immune response against a cancer cell;
preferably, the antigen is a tumor antigen or an immunogenic fragment thereof selected from the group consisting of: MelanA/MART1, cancer-germline antigens, gp100, tyrosinase, CEA, PSA, Her-2/neu, survivin, telomerase, and an immunogenic fragment thereof;
preferably, the cancer is selected from the group consisting of prostate cancer, non-small cell lung cancer, small cell lung cancer, renal cell carcinoma, brain cancer, melanoma, acute myeloid leukemia, pancreatic cancer, colorectal cancer, squamous cell carcinoma of the head and neck, squamous cell carcinoma of the skin, adenoid cystic carcinoma, glioblastoma, breast cancer, mesothelioma, ovarian cancer, glioma, bladder cancer, liver cancer, bone cancer, bone marrow cancer, stomach cancer, thyroid cancer, lymphoma, cervical cancer, endometrial cancer, laryngeal cancer, and acute lymphoblastic leukemia.

12. A composition comprising the mRNA of any one of claims 1 to 11.

13. The composition according to claim 12, wherein the composition further comprises a pharmaceutically acceptable carrier,
preferably, the pharmaceutically acceptable carrier is a lipid nanoparticle (LNP), a polymer material or an inorganic nanoparticle; and
preferably, the pharmaceutically acceptable carrier is cationic lipoplex (LPX), lipid polyplex (LPP), polymer nanoparticle (PNP), inorganic nanoparticle (INP) or cationic nanoemulsion (CNE).

14. A method for preventing or treating an infection with a pathogenic microorganism or a tumor, wherein the method comprises a step of administering the mRNA of any one of claims 1 to 11 or the composition of claim 12 or 13 to a subject.

15. The method according to claim 14, wherein the subject is a human or an animal,
preferably, the animal is cow, sheep, cat, canine, horse, rabbit, monkey, mouse, rat, alpaca or camel;
preferably, the subject is an immunocompromised human or animal;
preferably, the subject suffers from chronic lung disease, chronic obstructive pulmonary disease or asthma; and
preferably, the patient suffers from an underlying disease selected from the group consisting of heart disease, diabetes or lung disease.

16. Use of the mRNA of any one of claims 1 to 11 or the composition of claim 12 or 13 in preparation of a medicament or a kit for diagnosing, preventing or treating an infection with a pathogenic microorganism or a tumor in a subject,
the subject is a human or an animal,
preferably, the animal is cow, sheep, cat, canine, horse, rabbit, monkey, mouse, rat, alpaca or camel;
preferably, the subject is an immunocompromised human or animal;
preferably, the subject suffers from chronic lung disease, chronic obstructive pulmonary disease or asthma; and
preferably, the patient suffers from an underlying disease selected from the group consisting of heart disease, diabetes or lung disease.
